(19) Europäisches Patentamt
European Patent Office
Office européen des brevets

(11) EP 2 427 461 B1

(12) **EUROPÄISCHE PATENTSCHRIFT**

(45) Veröffentlichungstag und Bekanntmachung des Hinweises auf die Patenterteilung:
**09.10.2013 Patentblatt 2013/41**

(21) Anmeldenummer: **10714187.1**

(22) Anmeldetag: **08.04.2010**

(51) Int Cl.:
***C07D 471/04*** *(2006.01)* ***A61K 31/437*** *(2006.01)*
***A61P 35/00*** *(2006.01)*

(86) Internationale Anmeldenummer:
**PCT/EP2010/002207**

(87) Internationale Veröffentlichungsnummer:
**WO 2010/127754 (11.11.2010 Gazette 2010/45)**

(54) **3-([1,2,3]TRIAZOL-4-YL)-PYRROLO[2,3-B]PYRIDINDERIVATIVE**

3-([1,2,3]TRIAZOL-4-YL)-PYRROLO[2,3-B]PYRIDINE DERIVATIVES

DÉRIVÉS DE 3-([1,2,3]TRIAZOL-4-YL)-PYRROLO[2,3-B]PYRIDINE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **05.05.2009 DE 102009019962**

(43) Veröffentlichungstag der Anmeldung:
**14.03.2012 Patentblatt 2012/11**

(73) Patentinhaber: **Merck Patent GmbH**
**64293 Darmstadt (DE)**

(72) Erfinder:
- **DORSCH, Dieter**
  **64372 Ober-Ramstadt (DE)**
- **WUCHERER-PLIETKER, Margarita**
  **64291 Darmstadt (DE)**
- **MUELLER, Thomas, J., J.**
  **40591 Duesseldorf (DE)**
- **MERKUL, Eugen**
  **40591 Duesseldorf (DE)**

(56) Entgegenhaltungen:
**WO-A1-2005/105788 WO-A1-2009/054941**
**WO-A2-2009/003999**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 427 461 B1

## Beschreibung

Die Erfindung betrifft Verbindungen der Formel I

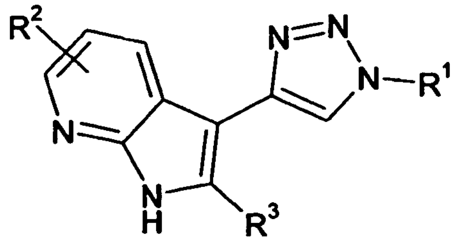

I

worin

R1 - C ($R^3$) ($R^4$)- Ar oder C ($R^3$) ($R^4$)- Het,
R2 H, A oder- [C $(R^3)_2]_n$- Het',
R3 H oder A,
R4 H, -[C $(R^3)_2]_nOR^3$ oder- (C $(R^3)_2]_nCOOR^3$,
A unverzweigtes oder verzweigtes Alkyl mit 1- 6 C- Atomen, worin eine $CH_2$- Gruppe durch ein O-, N oder S- Atom und/ oder auch 1- 7 H- Atome durch F ersetzt sein können,
Ar unsubstituiertes oder ein- oder zweifach durch Hal, -[C $(R^3)_2]_nOR^3$, $COOR^3$, CN und/ oder A substituiertes Phenyl,
Het einen unsubstituierten oder ein- oder zweifach durch A und/ oder Hal substituierten ein- oder zweikernigen gesättigten aromatischen Heterocyclus mit 1 bis 4 N-, und/ oder O- und/ oder S- Atomen,
Het' einen ein- oder zweikernigen aromatischen Heterocyclus mit 1 bis 4 N-, O- und/ oder S- Atomen, der unsubstituiert ist oder ein- oder zweifach durch A und/ oder [C $(R^3)_2]_nHet^1$ substituiert sein kann,
Het1 einen einkernigen gesättigten Heterocyclus mit 1 bis 2 N und/ oder O- Atomen, der ein- oder zweifach durch A sein kann,
Hal F, Cl, Br oder I
n 0, 1 oder 2,

bedeuten,
sowie ihre pharmazeutisch verwendbaren Salze, Tautomere und Stereoisomere, einschließlich deren Mischungen in allen Verhältnissen.

Die erfindungsgemäßen Verbindungen der Formel I umfassen auch deren pharmazeutisch verwendbaren Derivate und Solvate.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze und/oder Solvate bei guter Verträglichkeit sehr wertvolle pharmakologische Eigenschaften besitzen.

Insbesondere zeigen sie eine inhibierende Wirkung der Zellproliferation/ Zellvitalität als Antagonisten oder Agonisten. Die erfindungsgemäßen Verbindungen können daher zur Bekämpfung und/oder Behandlung von Tumoren, Tumorwachstum und/oder Tumormetastasen verwendet werden. Die antiproliferative Wirkung kann in einem Proliferationsassay/ Vitalitätsassay getestet werden.

Dementsprechend werden die erfindungsgemäßen Verbindungen oder ein pharmazeutisch unbedenkliches Salz davon für die Behandlung von Krebs verabreicht, einschließlich solider Karzinome, wie zum Beispiel Karzinome (z. B. der Lungen, des Pankreas, der Schilddrüse, der Harnblase oder des Kolons), myeloische Erkrankungen (z. B. myeloische Leukämie) oder Adenome (z. B. villöses Kolonadenom).

Zu den Tumoren zählen weiterhin die Monozytenleukämie, Hirn-, Urogenital-, Lymphsystem-, Magen-, Kehlkopf- und Lungenkarzinom, darunter Lungenadenokarzinom und kleinzelliges Lungenkarzinom, Bauchspeicheldrüsen- und/oder Brustkarzinom.

Die Verbindungen sind ferner nützlich bei der Behandlung der durch HIV-1 (Human Immunodeficiency Virus Typ 1) induzierten Immunschwäche.

Als krebsartige hyperproliferative Erkrankungen sind Hirnkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischer Krebs, Schilddrüsenkrebs, Lymphome, chronische Leukämie und akute Leukämie anzusehen. Insbesondere krebsartiges Zellwachstum ist eine Erkrankung, die ein Ziel der vorliegenden Erfindung darstellt. Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Es kann gezeigt werden, dass die erfindungsgemäßen Verbindungen antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation/ Vitalität wird durch Verabreichung der erfindungsgemäßen Verbindungen vor Entwicklung der evidenten Krankheit erreicht, z. B. zur Verhinderung des Tumorwachstums. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer inkubiert, die ausreicht, um den aktiven Mitteln zu ermöglichen, Zelltod zu induzieren oder Zellproliferation, Zellvitalität oder Migration zu inhibieren, gewöhnlich zwischen ungefähr einer Stunde und einer Woche. Zum Testen in vitro können kultivierte Zellen aus einer Biopsieprobe verwendet werden. Die Menge nach der Behandlung zurückbleibenden Zellen werden dann bestimmt. Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Es gibt viele mit einer Deregulation der Zellproliferation und des Zelltods (Apoptose) einhergehende Erkrankungen. Die Leiden von Interesse schließen die folgenden Leiden ein, sind aber nicht darauf beschränkt. Die erfindungsgemäßen Verbindungen sind nützlich bei der Behandlung einer Reihe verschiedener Leiden, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

Die Verbindungen der Formel I, wirken auch als Regulatoren, Modulatoren oder Inhibitoren von Proteinkinasen, insbesondere des Typs Serin/Threonin-Kinase, zu denen unter anderem die Phosphoinositid-abhängige Kinase 1 (PDK 1) gehören. Eine gewisse Wirkung zeigen die erfindungsgemäßen Verbindungen bei der Inhibierung der Serin/Threonin-Kinasen PDK1, IKKε und TBK1.

PDK1 phosphoryliert und aktiviert eine Untergruppe der AGC Proteinkinasen-Familie, umfassend PKB, SGK, S6K und PKC Isoformen. Diese Kinasen sind an dem PI3K Signalübertragungsweg beteiligt und kontrollieren grundlegende zelluläre Funktionen wie Überleben, Wachstum und Differenzierung. PDK1 ist somit ein bedeutender Regulator diverser metabolischer, proliferativer und Lebenserhaltungs-Effekte.

In der WO 2008/079988 A2 sind Chinazolinderivate als PDK1-Inhibitoren zur Krebsbekämpfung beschrieben.

In der WO 2008/112217 A1 sind Benzonaphthyridinderivate als PDK1-Inhibitoren zur Krebsbekämpfung beschrieben.

Pyridinonylderivate kennt man als PDK1-Inhibitoren zur Krebsbekämpfung aus der WO 2008/005457.

Pyrrolo- pyridin- kinase- modulatoren zur Krebsbekämpfung sind in WO WO 2008/124849 beschrieben.

In der WO 2006/106326 A1 und WO 2008/156726 A1 sind andere heterocyclische Verbindungen als PDK1-Inhibitoren zur Krebsbekämpfung beschrieben.

In der WO 2009/054941 A1 sind Pyrrolopyridin-derivate als PDK1-Inhibitoren zur Krebsbekämpfung beschrieben.

IKKε und TBK1 sind Serin/Threonin Kinasen die hohe Homologien untereinander sowie zu anderen IkB-Kinasen aufweisen. Beide Kinasen spielen eine integrale Role für das angebore, immanente Immunsystem. Dopplesträngige RNA-Viren werden durch die Toll-like Rezeptoren 3 und 4, sowie die RNA-Helicasen RIG-I and MDA-5 erkannt und

führen zu einer Aktivierung der TRIF-TBK1/IKKε-IRF3 Signalkaskade, was zu einer Typ I Interferon-Antwort führt.

Boehm und Kollegen beschrieben 2007 IKKε als ein neuartiges Brustkrebsonkogen [J.S. Boehm et al., Cell 129, 1065-1079, 2007]. 354 Kinasen wurden auf Ihre Fähigkeit hin untersucht gemeinschaftlich mit einer aktivierten Form der MAPK Kinase Mek, den Ras -transformierenden Phänotyp zu rekapitulieren. IKKε wurde hierbei als ein kooperatives Onkogen identifiziert.

Darüberhinaus konnten die Autoren zeigen, dass IKBKE in zahlreichen Brustkrebszelllinien und Tumorproben amplifiziert und überexpremiert vorliegt. Die Verminderung der Genexpression mittels RNA interference in Brustkrebszellen induziert Apoptosis und beeinträchtigt deren Proliferation. Eddy und Kollegen kamen 2005 zu ähnlichen Befunden, was die Bedeutung von IKKε in Brustkrebserkrankungen unterstreicht [S.F.Eddy et al., Cancer Res. 2005; 65 (24), 11375-11383].

Über einen protumorigenen Effekt von TBK1 wurde erstmals 2006 berichtet. Korherr und Kollegen identifizierten in einem Screening einer 251000 cDNA umfassenden Genbibliothek mit TRIF, TBK1 und IRF3 gleich drei Gene, die typischerweise in der angeborenen Immunabwehr involviert sind, als proangiogene Faktoren [C.Korherr et al., PNAS, 103, 4240-4245, 2006].

Chien und Kollegen publizierten 2006 [Y.Chien et al., Cell 127, 157-170, 2006], dass TBK1-/- Zellen nur bedingt mit oncogenem Ras transformierbar sind, was eine Involvierung von TBK1 bei der Ras-vermittelten Transformation nahelegt. Desweiteren konnten Sie zeigen, dass ein RNAi vermittelter knock down von TBK1 Apoptose in MCF-7 und Panc-1 Zellen auslöst. Kürzlich publizierten Barbie und Kollegen, dass TBK1 in zahlreichen Krebszellinien mit mutierten K-Ras von essentieller Bedeutung ist, was nahelegt, dass eine TBK1 Intervention in entsprechenden Tumoren von therapeutischer Bedeutung sein könnte [D.A.Barbie et al., Nature Letters 1-5, 2009].

Durch Proteinkinasen hervorgerufene Erkrankungen sind durch eine anomale Aktivität oder Hyperaktivität solcher Proteinkinasen gekennzeichnet. Anomale Aktivität betrifft entweder: (1) die Expression in Zellen, die gewöhnlich diese Proteinkinasen nicht exprimieren; (2) erhöhte Kinasen-Expression, die zu unerwünschter Zellproliferation, wie Krebs, führt; (3) erhöhte Kinasen-Aktivität, die zu unerwünschter Zellproliferation, wie Krebs, und/oder zu Hyperaktivität der entsprechenden Proteinkinasen führt. Hyperaktivität bezieht sich entweder auf eine Amplifikation des Gens, das eine bestimmte Proteinkinase codiert, oder die Erzeugung eines Aktivitäts-Spiegels, der mit einer Zellproliferationserkrankung korreliert werden kann (d.h. mit steigendem Kinase-Spiegel steigt die Schwere eines oder mehrerer Symptome der Zellproliferationserkrankung) die biologische Verfügbarkeit einer Proteinkinase kann auch durch das Vorhandensein oder Fehlen eines Satzes von Bindungsproteinen dieser Kinase beeinflusst werden.

Die wichtigsten Krebsarten, die unter Verwendung einer erfindungsgemäßen Verbindung behandelt werden können, umfassen Kolorektalkrebs, kleinzelligen Lungenkrebs, nicht-kleinzelligen Lungenkrebs, das multiple Myelom sowie das Nierenzellkarzinom und das Endometriumkarzinom, bersonders auch Krebsarten, in denen PTEN mutiert ist, u. a. Brustkrebs, Prostata-Krebs und Glioblastom.

Zudem können die erfindungsgemäßen Verbindungen verwendet werden, um bei gewissen existierenden Krebs-Chemotherapien und -bestrahlungen additive oder synergistische Effekte zu erzielen und/oder, um die Wirksamkeit gewisser existierender Krebs-Chemo -therapien und -bestrahlungen wiederherzustellen.

Unter Verbindungen der Formel I versteht man auch die Hydrate und Solvate dieser Verbindungen, ferner pharmazeutisch verwendbare Derivate. Gegenstand der Erfindung sind auch die optisch aktiven Formen (Stereoisomeren), Salze, die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate. Unter pharmazeutisch verwendbaren Derivaten versteht man z.B. die Salze der erfindungsgemäßen Verbindungen.

Hierzu gehören auch bioabbaubare Polymerderivate der erfindungsgemäßen Verbindungen, wie dies z. B. in Int. J. Pharm. 115, 61-67 (1995) beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:

verbesserte Heilbehandlung, Heilung, Prävention oder Beseitigung einer Krankheit, eines Krankheitsbildes, eines Krankheitszustandes, eines Leidens,
einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung ist auch die Verwendung von Mischungen der Verbindungen der Formel I, z.B. Gemische zweier Diastereomerer z.B. im Verhältnis 1:1, 1:2, 1:3, 1:4, 1:5, 1:10, 1:100 oder 1:1000.

Besonders bevorzugt handelt es sich dabei um Mischungen stereoisomerer Verbindungen.

Gegenstand der Erfindung sind die Verbindungen der Formel I und ihre Salze sowie ein Verfahren zur Herstellung von Verbindungen der Formel I sowie ihrer pharmazeutisch verwendbaren Salze, Tautomeren und Stereoisomeren, dadurch gekennzeichnet, daß man aus einer Verbindung der Formel II

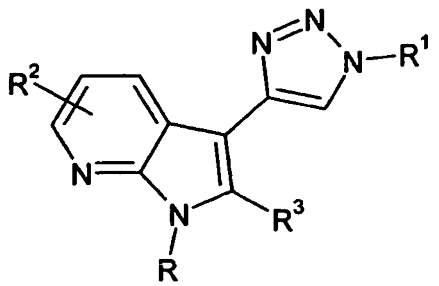

II ,

worin R eine Indolschutzgruppe bedeutet,
die Indolschutzgruppe abspaltet,
und/oder eine Base oder Säure der Formel I in eines ihrer Salze umwandelt.

Vor- und nachstehend haben die Reste $R^1$, $R^2$ und $R^3$ die bei der Formel I angegebenen Bedeutungen, sofern nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C- Atome. A bedeutet vorzugsweise Methyl, weiterhin Ethyl, Propyl, Isopropyl, Butyl, Isobutyl, sek.- Butyl oder tert.- Butyl, ferner auch Pentyl, 1-, 2- oder 3- Methylbutyl, 1, 1- , 1, 2- oder 2, 2- Dimethylpropyl, 1- Ethylpropyl, Hexyl, 1- , 2- , 3- oder 4- Methylpentyl, 1, 1- , 1, 2- , 1, 3- , 2, 2- , 2, 3- oder 3, 3- Dimethylbutyl, 1- oder 2- Ethylbutyl, 1- Ethyl- 1- methylpropyl, 1- Ethyl- 2- methylpropyl, 1, 1, 2- oder 1, 2, 2- Trimethylpropyl, weiter bevorzugt z.B. Trifluormethyl.

A bedeutet ganz besonders bevorzugt Alkyl mit 1, 2, 3, 4, 5 oder 6 C- Atomen, vorzugsweise Methyl, Ethyl, Propyl, Isopropyl, Butyl, Isobutyl, sek.- Butyl, tert.- Butyl, Pentyl, Hexyl, Trifluormethyl, Pentafluorethyl oder 1, 1, 1- Trifluorethyl.

In A kann auch eine $CH_2$-Gruppe durch ein N, O- oder S-Atom ersetzt sein. So bedeutet A z.B. auch 2-Methoxyethyl oder 2-Hydroxyethyl.

A bedeutet weiterhin vorzugsweise unverzweigtes oder verzweigtes Alkyl mit 1-6 C-Atomen, worin eine $CH_2$-Gruppe durch ein O-, N oder S-Atom und/oder auch 1-7 H-Atome durch F ersetzt sein können.

Ar bedeutet z.B. Phenyl, o-, m- oder p- Tolyl, o-, m- oder p- Ethylphenyl, o-, m- oder p- Propylphenyl, o-, m- oder p- Isopropylphenyl, o-, m- oder p- tert.- Butylphenyl, o-, m- oder p- Trifluormethylphenyl, o-, m- oder p- Fluorphenyl, o-, m- oder p- Bromphenyl, o-, m- oder p- Chlorphenyl, o-, m- oder p- Hydroxyphenyl, o-, m- oder p- Methoxyphenyl, o-, m- oder p- Carboxyphenyl, o-, m- oder p- Methoxycarbonylphenyl, o-, m- oder p- Ethoxycarbonylphenyl, o-, m- oder p- Cyanphenyl.

weiter bevorzugt 2, 3-, 2, 4-, 2, 5-, 2, 6-, 3, 4- oder 3, 5- Difluorphenyl, 2, 3-, 2, 4-, 2, 5-, 2, 6-, 3, 4- oder 3, 5- Dichlorphenyl, 2, 3-, 2, 4-, 2, 5-, 2, 6-, 3, 4- oder 3, 5- Dibromphenyl, p- Iodphenyl, 4- Fluor- 3- chlorphenyl oder 2- Fluor- 4- bromphenyl.

Ar bedeutet vorzugsweise unsubstituiertes oder ein- oder zweifach durch Hal, $-[C(R^3)_2]_nOR^3$, $COOR^3$, CN und/ oder A substituiertes Phenyl.

Het bedeutet, ungeachtetet weiterer Substitutionen, z.B. 2- oder 3- Furyl, 2- oder 3- Thienyl, 1-, 2- oder 3- Pyrrolyl, 1-, 2, 4- oder 5- Imidazolyl, 1-, 3-, 4- oder 5- Pyrazolyl, 2-, 4- oder 5- Oxazolyl, 3-, 4- oder 5- Isoxazolyl, 2-, 4- oder 5- Thiazolyl, 3-, 4- oder 5- Isothiazolyl, 2-, 3- oder 4- Pyridyl, 2-, 4-, 5- oder 6- Pyrimidinyl, weiterhin bevorzugt 1, 2, 3- Triazol- 1-, - 4- oder- 5- yl, 1, 2, 4- Triazol- 1-- 3- oder 5- yl, 1- oder 5- Tetrazolyl, 1, 2, 3- Oxadiazol- 4- oder- 5- yl, 1, 2, 4- Oxadi- azol- 3- oder- 5- yl, 1, 3, 4- Thiadiazol- 2- oder- 5- yl, 1, 2, 4- Thiadiazol- 3- oder- 5- yl, 1, 2, 3- Thiadiazol- 4- oder- 5- yl, 3- oder 4- Pyridazinyl, Pyrazinyl, 1-, 2-, 3-, 4-, 5-, 6- oder 7- Indolyl, 4- oder 5- Isoindolyl, 1-, 2-, 4- oder 5- Benzimidazoly, 1-, 2-, 3-, 4-, 5-, 6- oder 7- Indazolyl, 1-, 3-, 4-, 5-, 6- oder 7- Benzopyrazolyl, 2-, 4-, 5-, 6- oder 7- Benzoxazolyl, 3-, 4-, 5-, 6- oder 7- Benzisoxazolyl, 2-, 4-, 5-, 6- oder 7- Benzothiazolyl, 2-, 4-, 5-, 6- oder 7- Benzisothiazolyl, 4-, 5-, 6- oder 7- Benz- 2, 1, 3- oxadiazolyl, 2-, 3-, 4-, 5-, 6-, 7- oder 8- Chinolyl, 1-, 3-, 4-, 5-, 6-, 7- oder 8- Isochinolyl, 3-, 4-, 5-, 6-, 7- oder 8- Cinnolinyl, 2-, 4-, 5-, 6-, 7- oder 8- Chinazolinyl, 5- oder 6- Chinoxalinyl, 2-, 3-, 5-, 6-, 7- oder 8- 2H- Benzo [1, 4]- oxazinyl, weiter bevorzugt 1, 3- Benzodioxol- 5- yl, 1, 4- Benzodioxan- 6- yl, 2, 1, 3- Benzothiadiazol- 4- oder- 5- yl oder 2, 1, 3- Benzoxadiazol- 5- yl.

Die heterocyclischen Reste können auch teilweise oder vollständig hydriert sein.

Unsubstituiertes Het kann also z. B. auch bedeuten 2, 3- Dihydro- 2-, - 3-, - 4- oder- 5- furyl, 2, 5- Dihydro- 2-, - 3-, - 4- oder 5- furyl, Tetrahydro- 2- oder- 3- furyl, 1, 3- Dioxolan- 4- yl, Tetrahydro- 2- oder- 3- thienyl, 2, 3- Dihydro- 1-, - 2-, - 3-, - 4- oder- 5- pyrrolyl, 2, 5- Dihydro- 1-, - 2-, - 3-, - 4- oder- 5- pyrrolyl, 1-, 2- oder 3- Pyrrolidinyl, Tetrahydro- 1-, - 2- oder- 4- imidazolyl, 2, 3- Dihydro- 1-, - 2-, - 3-, - 4- oder- 5- pyrazolyl, Tetrahydro- 1-, - 3- oder- 4- pyrazolyl, 1, 4- Dihydro- 1-, - 2-, - 3- oder- 4- pyridyl, 1, 2, 3, 4- Tetrahydro- 1-, - 2-, - 3-, - 4-, - 5- oder- 6- pyridyl, 1-, 2-, 3- oder 4- Piperidinyl, 2-, 3- oder 4- Morpholinyl, Tetrahydro- 2-, - 3- oder- 4- pyranyl, 1, 4- Dioxanyl, 1, 3- Dioxan- 2-, - 4- oder- 5- yl, Hexahydro- 1-, - 3- oder- 4- pyridazinyl, Hexahydro- 1-, - 2-, - 4- oder- 5- pyrimidinyl, 1-, 2- oder 3- Piperazinyl, 1, 2, 3, 4- Tetrahydro- 1-, - 2-, - 3-, - 4-, - 5-, - 6-, - 7- oder- 8- chinolyl, 1, 2, 3, 4- Tetrahydro- 1-, - 2-, - 3-, - 4-, - 5-, - 6-, - 7- oder- 8- isochinolyl, 2-, 3-, 5-, 6-, 7- oder 8- 3, 4- Dihydro- 2H- benzo [1, 4] oxazinyl, weiter bevorzugt 2, 3- Methylendioxyphenyl, 3, 4- Methylendioxyphenyl, 2, 3- Ethylendioxyphenyl, 3, 4- Ethylendioxyphenyl, 3, 4- (Difluormethylendioxy) phenyl, 2, 3- Dihydro- benzofuran- 5- oder 6- yl, 2, 3- (2- Oxo- methylendioxy)- phenyl oder auch 3, 4- Dihydro- 2H- 1, 5- benzodioxepin- 6- oder- 7- yl, ferner bevorzugt 2, 3- Dihydro- benzofuranyl oder 2, 3- Dihydro- 2- oxo- furanyl.

Het bedeutet weiterhin vorzugsweise einen unsubstituierten oder ein- oder zweifach durch A und/oder Hal substituierten ein- oder zweikernigen gesättigten aromatischen Heterocyclus mit 1 bis 4 N-, und/oder O- und/oder S-Atomen.

Het bedeutet ganz besonders bevorzugt unsubstituiertes oder ein- oder zweifach durch A und/ oder Hal substituiertes Furyl, Thienyl, Pyrrolyl, Imidazolyl, Pyrazolyl, Oxazolyl, Isoxazolyl, Thiazolyl, Pyridyl, Pyrimidinyl, Triazolyl, Tetrazolyl, Thiadiazol, Pyridazinyl, Pyrazinyl, Indolyl, Isoindolyl, Benzimidazolyl, Indazolyl, Chinolyl, 1, 3- Benzodioxolyl, Pyrrolidinyl, Tetrahydro- imidazolyl, Tetrahydro- pyrazolyl, Piperidinyl, Morpholinyl, Piperazinyl, Oxazolidinyl oder Isoxazolidin.

Het' hat, ungeachtet weiterer Substitutionen, vorzugsweise die Bedeutungen wie für Het angegeben.

Het' bedeutet weiterhin vorzugsweise einen ein- oder zweikernigen aromatischen Heterocyclus mit 1 bis 4 N-, O- und/ oder S- Atomen, der unsubstituiert ist oder ein- oder zweifach durch A und/ oder $[C(R^3)_2]_nHet^1$ substituiert sein kann.

Het' bedeutet ganz besonders bevorzugt unsubstituiertes oder ein- oder zweifach durch A und/ oder $[C(R^3)_2]_nHet^1$ substituiertes Furyl, Thienyl, Pyrrolyl, Imidazolyl, Pyrazolyl, Oxazolyl, Isoxazolyl, Thiazolyl, Pyridyl, Pyrimidinyl, Triazolyl, Tetrazolyl, Thiadiazol, Pyridazinyl, Pyrazinyl, Indolyl, Isoindolyl, Benzimidazolyl, Indazolyl, Chinolyl oder 1, 3- Benzodioxolyl.

$Het^1$ bedeutet vorzugsweise einen einkernigen gesättigten Heterocyclus mit 1 bis 2 N und/ oder O- Atomen, der ein- oder zweifach durch A substituiert sein kann.

$Het^1$ bedeutet ganz besonders bevorzugt unsubstituiertes oder ein- oder zweifach durch A substituiertes Pyrrolidinyl, Tetrahydro- imidazolyl, Tetrahydropyrazolyl, Piperidinyl, Morpholinyl, Piperazinyl, Oxazolidinyl oder Isoxazolidinyl.

$R^1$ bedeutet vorzugsweise- $C(R^3)(R^4)$- Ar oder $C(R^3)(R^4)$- Het.

$R^2$ bedeutet H, A oder- $[C(R^3)_2]_n$- Het'.

$R^3$ bedeutet vorzugsweise H, Methyl, Ethyl, Propyl oder Butyl.

$R^4$ bedeutet vorzugsweise H, $-[C(R^3)_2]_nOR^3$ oder- $[C(R^3)_2]_nCOOR^3$.

Hal bedeutet vorzugsweise F, Cl oder Br, aber auch I, besonders bevorzugt F oder Cl.

Für die gesamte Efindung gilt, daß sämtliche Reste, die mehrfach auftreten, gleich oder verschieden sein können, d.h. unabhängig voneinander sind.

Die Verbindungen der Formel I können ein oder mehrere chirale Zentren besitzen und daher in verschiedenen stereoisomeren Formen vorkommen. Die Formel I umschließt alle diese Formen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben- Weyl, Methoden der organischen Chemie, Georg- Thieme- Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Verbindungen der Formel I können vorzugsweise erhalten werden, indem man aus Verbindungen der Formel II die Indolschutzgruppe abspaltet.

Die Umsetzung erfolgt in einem inerten Lösungsmittel und erfolgt in der Regel in Gegenwart eines Alkali- oder Erdalkalimetall-hydroxids, -carbonats oder - bicarbonats oder eines anderen Salzes einer schwachen Säure der Alkali- oder Erdalkalimetalle, vorzugsweise des Kaliums, Natriums, Calciums oder Cäsiums.

Die Reaktionszeit liegt je nach den angewendeten Bedingungen zwischen einigen Minuten und 14 Tagen, die Reaktionstemperatur zwischen etwa -15° und 150°, normalerweise zwischen 10° und 100°, besonders bevorzugt zwischen 15° und 80°C.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1, 2- Dichlorethan, Tetrachlorkohlenstoff, Chloroform oder Dichlormethan; Alkohole wie Methanol, Ethanol, Isopropanol, n- Propanol, n- Butanol oder tert.- Butanol; Ether wie Diethylether,

Diisopropylether, Tetrahydrofuran (THF) oder Dioxan; Glykolether wie Ethylenglykolmonomethyl- oder- monoethylether (Methylglykol oder Ethylglykol), Ethylenglykoldimethylether (Diglyme) ; Ketone wie Aceton oder Butanon; Amide wie Acetamid, Dimethylacetamid oder Dimethylformamid (DMF) ; Nitrile wie Acetonitril; Sulfoxide wie Dimethylsulfoxid (DMSO) ; Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Besonders bevorzugt sind Alkohole, wie z.B. Methanol.

Bevorzugte Indolschutzgruppen sind z.B. Sulfonylschutzgruppen, wie Tosyl oder Mesyl, ferner Schutzgruppen wie z.B. BOC.

Verbindungen der Formel II können vorzugsweise erhalten werden, indem man eine Verbindung der Formel III

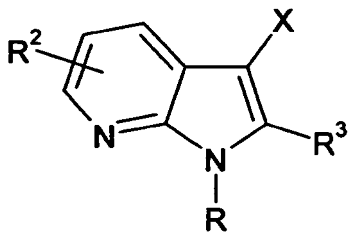

III

worin
$R^2$ und $R^3$ die in Anspruch 1 angegebenen Bedeutungen haben,
R eine Indolschutzgruppe,
X Cl, Br, I oder OTf,
bedeuten,

in einer Sonogashira-Reaktion vorzugsweise kupferkatalysiert mit Trimethylsilylacetylen und einem Übergangsmetallkatalysator umsetzt [Lit.: Rafael Chinchilla und Carmen Nájera, Chem. Rev. 2007, 107, 874-922], danach die Trimethylsilylgruppe abspaltet und anschließend in einer Azid-Alkin-Cycloaddition mit einer Verbindung der Formel IV

$N{=}N{=}N\text{-}R^1$ IV

worin $R^1$ die in Anspruch 1 angegebene Bedeutung hat,
umsetzt [Lit.: Morten Meldal und Christian Wenzel Tornøe, Chem. Rev., 2008, 108 (8), 2952-3015]. Die Verbindung IV kann auch durch Umsetzung eines Alkylhalogenids mit einem Alkaliazid hergestellt werden.

<u>Pharmazeutische Salze und andere Formen</u>

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen (III) -, Eisen (II) -, Lithium-, Magnesium-, Mangan (III) -, Mangan (II), Kalium-, Natrium- und Zinksalze, was jedoch keine Einschränkung darstellen soll. Bevorzugt unter den oben genannten Salzen sind Ammonium; die Alkalimetallsalze Natrium und Kalium, sowie die Erdalkalimetalsalze Calcium und Magnesium. Zu Salzen der Verbindungen der Formel I, die sich von pharmazeutisch unbedenklichen organischen nicht- toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N, N'- Dibenzylethylendiamin (Benzathin), Dicyclohexylamin, Diethanolamin, Diethylamin, 2- Diethylaminoethanol, 2- Dimethylaminoethanol, Ethanolamin, Ethylendiamin, N- Ethylmorpholin, N- Ethylpiperidin, Glucamin, Glucosamin, Histidin, Hydrabamin, Iso- propylamin, Lidocain, Lysin, Meglumin, N- Methyl- D- glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)- methylamin (Tromethamin), was jedoch keine Einschränkung darstellen soll.

Verbindungen der vorliegenden Erfindung, die basische stickstoffhaltige Gruppen enthalten, lassen sich mit Mitteln wie ($C_1$- $C_4$) Alkylhalogeniden, z.B. Methyl-, Ethyl-, Isopropyl- und tert.- Butylchlorid, -bromid und- iodid; Di ($C_1$- $C_4$) Alkylsulfaten, z.B. Dimethyl-, Diethyl- und Diamylsulfat; ($C_{10}$- $C_{18}$) Alkylhalogeniden, z.B. Decyl-, Dodecyl-, Lauryl-, Myristyl- und Stearylchlorid, -bromid und- iodid; sowie Aryl- ($C_1$- $C_4$) Alkylhalogeniden, z.B. Benzylchlorid und Phenethylbromid, quarternisieren. Mit solchen Salzen können sowohl wasserals auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N, N'- Dibenzylethylendiamin, Chlorprocain, Cholin, Diethanolamin, Ethylendiamin, N- Methyl- D- glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel I und/oder ihre pharmazeutisch verwendbaren Salze, Tautomeren und Stereoisomeren, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem

behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff (en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl- in- Wasser- Flüssigemulsionen oder Wasser- in- Öl-Flüssigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I sowie Salze, Tautomeren und Stereoisomeren davon lassen sich auch in Form von Liposomenzuführsystemen, wie z.B. kleinen unilamellaren Vesikeln, großen unilamellaren Vesikein und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie die Salze, Tautomeren und Stereoisomeren davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl- in- Wasser- Cremebasis oder einer Wasser- in- Öl- Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Vemeblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von

dem behandeln den Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es läßt sich annehmen, daß ähnliche Dosierungen für die Behandlung der anderen, oben erwähnten Krankheitszustände geeignet sind.

Gegenstand der Erfindung sind ferner Arzneimittel enthaltend mindestens eine Verbindung der Formel I und/oder ihre pharmazeutisch verwendbaren Salze und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von

(a) einer wirksamen Menge an einer Verbindung der Formel I und/oder ihrer pharmazeutisch verwendbaren Salze und Stereoisomere, einschließlich deren Mischungen in allen Verhältnissen,
und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Salze und Stereoisomere, einschließlich deren Mischungen in allen Verhältnissen, und einer wirksamen Menge eines weiteren Arzneimittelwirkstoffs gelöst oder in lyophilisierter Form vorliegt.

## VERWENDUNG

Die vorliegenden Verbindungen eignen sich als pharmazeutische Wirkstoffe für Säugetiere, insbesondere für den Menschen, bei der Behandlung und Bekämpfung von Krebserkrankungen.

Gegenstand der Erfindung sind weiterhin die Verbindungen der Formel I gemäß Anspruch 1-2 sowie ihrer pharmazeutisch verwendbaren Salze, Tautomeren und Stereoisomeren, einschließlich deren Mischungen in allen Verhältnissen, zur Verwendung zur Behandlung von Tumoren, Tumorwachstum, Tumormetastasen und/oder AIDS.

Die vorliegende Erfindung umfasst die Verwendung der Verbindungen der Formel I und/oder ihre physiologisch unbedenklichen Salze, Tautomeren und Stereoisomeren zur Herstellung eines Arzneimittels zur Behandlung oder Vorbeugung von Krebs. Bevorzugte Karzinome für die Behandlung stammen aus der Gruppe Hirnkarzinom, Urogenitaltraktkarzinom, Karzinom des lymphatischen Systems, Magenkarzinom, Kehlkopfkarzinom und Lungenkarzinom Darmkrebs. Eine weitere Gruppe bevorzugter Krebsformen sind Monozytenleukämie, Lungenadenokarzinom, kleinzellige Lungenkarzinome, Bauchspeicheldrüsenkrebs, Glioblastome und Brustkarzinom.

Ebenfalls umfasst ist die Verwendung der Verbindungen der Formel I und/oder ihre physiologisch unbedenklichen Salze, Tautomeren und Stereoisomeren zur Herstellung eines Arzneimittels zur Behandlung und/oder Bekämpfung einer durch Tumore bedingten Krankheit bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.

Insbesondere bevorzugt ist die Verwendung zur Behandlung einer Krankheit, wobei die Krankheit ein fester Tumor ist.

Der feste Tumor ist vorzugsweise ausgewählt aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopf und/oder der Lunge.

Der feste Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt ist die Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Gegenstand der Erfindung ist weiterhin die Verwendung der erfindungsgemäßen Verbindungen zur Behandlung von Knochen-Pathologien, wobei die Knochenpathologie aus der Gruppe Osteosarkom, Osteoarthritis und Rachitis

stammt.

Die Verbindungen der Formel I können auch gemeinsam mit anderen gut bekannten Therapeutika, die aufgrund ihrer jeweiligen Eignung für das behandelte Leiden ausgewählt werden, verabreicht werden.

Die vorliegenden Verbindungen eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl- Proteintransferasehemmer, HMG- CoA- Reduktase- Hemmer, HIV- Protease- Hemmer, Reverse- Transkriptase- Hemmer sowie weitere Angiogenesehemmer. Die vorliegenden Verbindungen eignen sich insbesondere zur gemeinsamen Anwendung mit Radiotherapie. "Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4- [7- (2, 2- Dimethyl- 1- oxopropoxy- 4- methyl- 2- [4- [2- (1- piperidinyl) ethoxy]- phenyl]- 2H- 1- benzopyran- 3- yl] phenyl- 2, 2- dimethylpropanoat, 4, 4'- Dihydroxybenzophenon- 2, 4- dinitrophenylhydrazon und SH646, was jedoch keine Einschränkung darstellen soll.

"Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5$\alpha$- Reduktase- Hemmer, Nilutamid, Flutamid, Bicalutamid, Liarozol und Abirateron- acetat.

"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13- cis- Retinsäure, 9- cis- Retinsäure, $\alpha$- Difluormethylornithin, ILX23- 7553, trans- N- (4'- Hydroxyphenyl) retinamid und N- 4- Carboxyphenylretinamid.

"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkaliernde Mittel, Mikrotubulin-Hemmer und Topoisomerase-Hemmer.

Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan- tosylat, Trofosfamid, Nimustin, Dibrospidium- chlorid, Pumitepa, Lobaplatin, Satraplatin, Profiromycin, Cisplatin, Irofulven, Dexifosfamid, cis- Amindichlor (2- methylpyridin) platin, Benzylguanin, Glufosfamid, GPX100, (trans, trans, trans)- bis- mu- (hexan- 1, 6- diamin)- mu- [diamin- platin (11) ] bis [diamin (chlor) platin (11) ]- tetrachlorid, Diarizidinylspermin, Arsentrioxid, 1- (11- Dodecylamino- 10- hydroxyundecyl)- 3, 7- dimethylxanthin, Zorubicin, Idarubicin, Daunorubicin, Bisantren, Mitoxantron, Pirarubicin, Pinafid, Valrubicin, Amrubicin, Antineoplaston, 3'- Desamino- 3'- morpholino- 13- desoxo- 10- hydroxycarminomycin, Annamycin, Galarubicin, Elinafid, MEN10755 und 4- Desmethoxy- 3- desamino- 3- aziridinyl- 4- methylsulfonyl- daunorubicin (siehe WO 00/50032), was jedoch keine Einschränkung darstellen soll.

Zu den Mikrotubulin- Hemmern zählen zum Beispiel Paclitaxel, Vindesin- sulfat, 3', 4'- Dideshydro- 4'- desoxy- 8'- norvincaleukoblastin, Docetaxol, Rhizoxin, Dolastatin, Mivobulin- isethionat, Auristatin, Cemadotin, RPR109881, BMS184476, Vinflunin, Cryptophycin, 2, 3, 4, 5, 6- pentafluor- N- (3- fluor- 4- methoxyphenyl) benzolsulfonamid, Anhydrovinblastin, N, N- dimethyl- L- valyl- L- valyl- N- methyl- L- valyl- L- prolyl- L- prolin- t- butylamid, TDX258 und BMS188797. Topoisomerase- Hemmer sind zum Beispiel Topotecan, Hycaptamin, Irinotecan, Rubitecan, 6- Ethoxypropionyl- 3', 4'- O- exo- benzyliden- chartreusin, 9- Methoxy- N, N- dimethyl- 5- nitropyrazolo [3, 4, 5- kl] acridin- 2- (6H) propanamin, 1- Amino- 9- ethyl- 5- fluor- 2, 3- dihydro- 9- hydroxy- 4- methyl- 1H, 12H- benzo [de]- pyrano [3', 4': b, 7] indolizino [1, 2b] chinolin- 10, 13 (9H, 15H)- dion, Lurtotecan, 7- [2- (N- Isopropylamino) ethyl]- (20S) camptothecin, BNP1350, BNPI1100, BN80915, BN80942, Etoposid- phosphat, Teniposid, Sobuzoxan, 2'- Dimethylamino- 2'- desoxy- etoposid, GL331, N- [2- (Dimethylamino) ethyl]- 9- hydroxy- 5, 6- dimethyl- 6H- pyrido [4, 3- b] carbazol- 1- carboxamid, Asulacrin, (5a, 5aB, 8aa, 9b)- 9- [2- [N- [2- (Dimethylamino) ethyl]- N- methylamino] ethyl]- 5- [4- hydroxy- 3, 5- dimethoxyphenyl]- 5, 5a, 6, 8, 8a, 9- hexohydrofuro (3', 4': 6, 7) naphtho (2, 3- d)- 1, 3- dioxol- 6- on, 2, 3- (Methylendioxy)- 5- methyl- 7- hydroxy- 8- methoxybenzo [c] phenanthridinium, 6, 9- Bis[(2- aminoethyl) amino] benzo [g] isochinolin- 5, 10- dion, 5- (3- Aminopropyl- amino)- 7, 10- dihydroxy- 2- (2- hydroxyethylaminomethyl)- 6H- pyrazolo [4, 5, 1- de]- acridin- 6- on, N- [1- [2 (Diethylamino) ethylamino]- 7- methoxy- 9- oxo- 9H- thioxan- then- 4- ylmethyl] formamid, N- (2- (Dimethylamino)- ethyl) acridin- 4- carboxamid, 6- [[2- (Dimethylamino)- ethyl] amino]- 3- hydroxy- 7H- indeno [2, 1- c] chinolin- 7- on und Dimesna.

Zu den "antiproliferativen Mitteln" zählen Antisense- RNA- und- DNA- Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabin- ocfosfat, Fosteabin- Natriumhydrat, Raltitrexed, Paltitrexid, Emitefur, Tiazofurin, Decitabin, Nolatrexed, Pemetrexed, Nelzarabin, 2'- Desoxy- 2'- methyliden- cytidin, 2'- Fluormethylen- 2'- desoxycytidin, N- [5- (2, 3- Dihydrobenzofuryl)- sulfonyl]- N'- (3, 4- dichlorphenyl) harnstoff, N6- [4- Desoxy- 4- [N2- [2 (E), 4 (E)- tetra- decadienoyl] glycylamino]- L- glycero- B- L- manno- heptopyranosyl] adenin, Aplidin, Ecteinascidin, Troxacitabine, 4- [2- Amino- 4- oxo- 4, 6, 7, 8- tetrahydro- 3H- pyrimidino [5, 4- b] [1, 4] thiazin- 6- yl- (S)- ethyl]-2, 5- thienoyl- L- glutaminsäure, Aminopterin, 5- Flurouracil, Alanosin, 11- Acetyl- 8- (carbamoyloxymethyl)- 4- formyl- 6- methoxy- 14- oxa- 1, 11- diazatetracyclo (7.4.1.0.0)- tetradeca- 2, 4, 6- trien- 9- ylessigsäureester, Swainsonin, Lometrexol, Dexrazoxan, Methioninase, 2'- cyan- 2'- desoxy- N4- palmitoyl- 1- B- D- Arabinofuranosylcytosin und 3- Aminopyridin- 2- carboxaldehyd- thiosemicarbazon. Die "antiproliferativen Mittel" beinhalten auch andere monoklonale Antikörper gegen Wachstumsfaktoren als bereits unter den "Angiogenese- Hemmern" angeführt wurden, wie Trastuzumab, sowie Tumorsuppressorgene, wie p53, die über rekombinanten virusvermittelten Gentransfer abgegeben werden können (siehe z.B. US- Patent Nr. 6, 069, 134) .

**Wirkungsnachweis von pharmakologischen Inhibitoren auf die Proliferation/Vtalität von Tumorzellen *in vitro***

**<u>1.0 Hintergrund</u>**

In der vorliegenden Versuchsbeschreibung wird die Hemmung der Tumorzellproliferation/ Tumorzellvitalität durch Wirkstoffe beschrieben.

Die Zellen werden in geeigneter Zelldichte in Mikrotiterplatten (96-well Format) ausgesät und die Testsubstanzen werden in Form einer Konzentrationreihe zugegeben. Nach vier weiteren Tagen der Kultivierung in serumhaltigem Medium kann die Tumorzellproliferation/ Tumorzellvitalität mittels eines Alamarblue-Testsystem bestimmt werden.

**<u>2.0 Versuchsdurchführung</u>**

**<u>2.1 Zellkultur</u>**

Beispielsweise käuflich erhältliche Colon- Carcinom- Zelllinien, Zelllinien des Eierstocks, Zellinien der Prostata oder Zelllinien der Brust etc.

Die Zellen werden in Medium kultiviert. In Abständen von mehreren Tagen werden die Zellen mit Hilfe von Trypsin-Lösung von den Kulturschalen abgelöst und in geeigneten Verdünnung in frischem Medium ausgesät. Die Zellen werden bei 37° Celsius und 10% $CO_2$ kultiviert.

**<u>2.2. Aussaat der Zellen</u>**

Eine definierte Zellzahl (z.B. 2000 Zellen) werden pro Kultur/ well in einem Volumen von 180µl Kulturmedium in Mikrotiterplatten (96 well Zellkulturplatten) mit einer Mehrkanalpipette ausgesät. Die Zellen werden anschließend in einem CO2-Brutschran (37°C und 10% CO2) kultiviert.

**<u>2.3. Zugabe der Testsubstanzen</u>**

Die Testsubstanzen werden beispielsweise in DMSO gelöst und anschließend in entsprechender Konzentration (gegebenenfalls einer Verdünnungsreihe) im Zellkulturmedium eingesetzt. Die Verdünnungs-stufen können je nach Effizienz der Wirkstoffe und gewünschter Spreizung der Konzentrationen angepasst werden. Die Testsubstanzen werden in entsprechenden Konzentrationen mit Zellkulturmedium versetzt. Die Zugabe der Testsubstanzen zu den Zellen kann am selben Tag wie die Aussat der Zellen erfolgen. Dazu wird aus der Vorverdünnungsplatte jeweils 20µl Substanzlösung in die Kulturen/wells gegeben. Die Zellen werden für weitere 4 Tage bei 37°Celsius und 10% $CO_2$ kultiviert.

<u>2.4. Messung der Farbreaktion</u>

Pro well werden jeweils 20µl AlamarBlue Reagenz gegeben und die Microtiterplatten werden beispielsweise für weitere sieben Stunden in einem CO2-Brutschrank (bei 37°C und 10% CO2) inkubiert. Die Platten werden an einem Reader mit einem Fluoreszenzfilter bei einer Wellenlänge von 540nm gemessen. Die Platten können direkt vor der Messung leicht geschüttelt werden.

**<u>3. Auswertung</u>**

Der Extinktionswert der Mediumkontrolle (keine Verwendung von Zellen und Testsubstanzen) wird von allen anderen Extinktionswerten subtrahiert. Die Kontrollen (Zellen ohne Testsubstanz) werden gleich 100 Prozent gesetzt und alle anderen Extinktionswerte hierzu in Beziehung gesetzt (beispielsweise in % der Kontrolle) ausgedrückt:

**Rechnung:**

$$\frac{100 * (\text{Wert mit Zellen und Testsubstanz - Wert der Mediumkontrolle})}{(\text{Wert mit Zellen - Wert der Mediumkontrolle})}$$

Die Bestimmung von $IC_{50}$ Werten (50%ige Hemmung) erfolgt mit Hilfe von Statistikprogrammen wie z.B. RS1.

**4.0 Test zur Inhibierung von PDK1**

Die Versuchsansätze werden in einem Flashplate-System mit 384 wells/Mikrotitrierplatte durchgeführt.

Pro well werden jeweils die PDK1- Probe $His_6$- PDK1 (□1- 50) (3.4 nM), das PDK1- Substrat Biotin- bA- bA- KTFCGTPEYLAPEVRREP- RILSEEEQEMFRDFDYIADWC (400 nM), 4 µM ATP (mit 0.2µCi $^{33}$P- ATP/ well) und die Testsubstanz in 50µl gebräuchlicher Versuchslösung für 60 Min bei 30°C inkubiert. Die Testsubstanzen werden in entsprechenden Konzentrationen (gegebenenfalls in einer Verdünnungsreihe) eingesetzt. Die Kontrolle wird ohne Testsubstanz durchgeführt. Die Reaktion wird mit gängigen Methoden gestoppt und gewaschen. Die Aktivität der Kinase wird über die eingebaute Radioaktivität im Topcount gemessen. Zur Bestimmung der unspezifischen Kinasereaktion (Leerwert) werden die Versuchsansätze in Gegenwart von 100 nM Staurosporin durchgeführt.

**5.0 Auswertung**

Die Radioaktivität (Zerfälle pro Minute) des Leerwerts (keine Verwendung von Testsubstanz in Gegenwart von Staurosporin) wird von allen anderen Radioaktivitätswerten substrahiert. Die Kontrollen (Kinaseaktivität ohne Testsubstanz) werden gleich 100 Prozent gesetzt und alle anderen Radioaktivitätswerte (nach Abzug des Leerwerts) hierzu in Beziehung gesetzt (beispielsweise in % der Kontrolle) ausgedrückt.

**Rechnung:**

$$\frac{100 * (\text{Wert der Kinaseaktivität mit Testsubstanz - Leerwert})}{(\text{Wert der Kontrolle - Leerwert})} = \%\ \text{der Kontrolle}$$

Die Bestimmung von $IC_{50}$ Werten (50%ige Hemmung) erfolgt mit Hilfe von Statistikprogrammen wie z.B. RS1. $IC_{50}$-Daten erfindungsgemäßer Verbindungen sind in Tabelle 1 angegeben.

| Material | Best. Nr. | Hersteller |
|---|---|---|
| Mikrotiterplatten für die Zellkultur (Nunclon Surface 96well Plate) | | 167008 Nunc |
| DMEM | P04-03550 | Pan Biotech |
| PBS (10x) Dulbecco | 14200-067 | Gibco |
| 96well Platten (Polypropylen) | 267334 | Nunc |
| AlamarBlue™ | BUF012B | Serotec |
| FCS | 1302 | Pan Biotech GmbH |
| Trypsin/EDTA Solution 10x | L 2153 | Biochrom AG |
| 75cm$^2$ Kulturflaschen | 353136 | BD Falcon |
| A2780 | 93112519 | ECACC |
| Colo205 | CCL222 | ATCC |
| MCF7 | HTB22 | ATCC |
| PC3 | CRL-1435 | ATCC |
| 384well Flash Platten | SMP410A001PK | Perkin Elmer |

APCI- MS (atmospheric pressure chemical ionization- mass spectrometry) $(M+H)^+$.

**Beschreibung der Methode zur zellulären Testung von PDK1-Kinase-Inhibitoren**

Der zelluläre Assay zur Bestimmung der PDK1 Kinase Aktivität wird als Luminex-Assay im 96-well Format durchgeführt. PC3 Zellen werden mit 20.000 Zellen pro well in 100 μl Medium (45% RPMI1460 / 45% Ham's F12 / 10% FCS) ausgesät und am folgenden Tag für 30 min mit einer seriellen Verdünnung der Prüfsubstanz (7 Konzentrationen) unter serumfreien Bedingungen inkubiert. Im Anschluss werden die Zellen mit 90 μl Lysepuffer (20mM Tris/HCl pH 8,0, 150mM NaCl, 1% NP40, 10% Glycerol, 1 % Phosphatase-Inhibitor I, 1 % Phosphatase-Inhibitor II, 0,1% Protease-Inhibitor Cocktail III, 0,01 % Benzonase) pro well lysiert, und die Lysate werden mittels Zentrifugation durch eine 96-well Filterplatte (0,65 μm) von unlöslichen Zellbestandteilen abgetrennt. Die Lysate werden üN bei 4°C mit Luminex-Beads, an die ein anti-total PKB Antikörper gekoppelt ist, unter Schütteln inkubiert. Am folgenden Tag erfolgt die Detektion durch Zugabe eines P-T308-PKB Antikörpers sowie eines speziesspezifischen PE-markierten Sekundär-antikörpers. Der Nachweis von P-T308-PKB erfolgt durch Messung im Luminex100 Gerät durch Bestimmung von 100 Ereignissen pro Kavität in 60 sec Messzeit. Als pharmakologischer Blank werden die erhaltenen Signale von Zellen, die mit 10 μM Staurosporin behandelt wurden, von allen anderen Ansätzen abgezogen. Als Kontrollwert der maximalen Phosphorylierung von PKB an T308 werden die Signale von Zellen, die nur mit dem Lösungsmittel (0,3% DMSO) behandelt wurden, verwendet. Die Werte der mit Prüfsubstanz behandelten Ansätze werden hiervon als Prozent von Kontrolle berechnet und IC50 Werte werden mittels RS1 ermittelt.

$IC_{50}$- Daten erfindungsgemäßer Verbindungen sind in Tabelle 1 angegeben.

IKKε - Kinase Test (IKKepsilon)

Der Kinaseassay wird als 384- well Flashplate assay durchgeführt. 1 nM IKKε, 800 nM biotinyliertes IκBα (19-42)- Peptid (Biotin- C6- C6- GLKKERLLDDRHDSGLDSMKDEE) und 10 μM ATP (mit 0, 3 μCi $^{33}P$- ATP/ well) werden in einem Gesamtvolumen von 50μl (10 mM MOPS, 10 mM Magnesiumacetat, 0, 1 mM EGTA, 1 mM Dithiothreitol, 0, 02 % Brij35, 0, 1 % BSA, 0, 1% BioStab, pH 7, 5) ohne oder mit Prüfsubstanz für 120 Min bei 30°C inkubiert. Die Reaktion wird mit 25μl 200 mM EDTA- Lösung gestoppt, nach 30 Min bei Raumtemperatur abgesaugt und die Wells 3mal mit 100 μl 0, 9%- ige NaCl- Lösung gewaschen. Der unspezifische Anteil der Kinasereaktion (Blank) wird mit 3 μM EMD 1126352 (BX- 795) bestimmt. Radioaktivität wird im Topcount gemessen. $IC_{50}$- Werte werden mit RS1 berechnet.

TBK1 - Kinase Test

Der Kinaseassay wird als 384- well Flashplate assay durchgeführt. 0, 6 nM TANK binding kinase (TBK1), 800 nM biotinyliertes MELK- derived peptide (Biotin- Ah- Ah- AKPKGNKDYHLQTCCGSLAYRRR) und 10 μM ATP (mit 0, 25 μCi $^{33}P$- ATP/ well) werden in einem Gesamtvolumen von 50μl (10 mM MOPS, 10 mM Magnesiumacetat, 0, 1 mM EGTA, 1 mM DTT, 0, 02 % Brij35, 0, 1 % BSA, pH 7, 5) ohne oder mit Prüfsubstanz für 120 Min bei 30°C inkubiert. Die Reaktion wird mit 25μl 200 mM EDTA- Lösung gestoppt, nach 30 Min bei Raumtemperatur abgesaugt und die Wells 3mal mit 100 μl 0, 9%- ige NaCl- Lösung gewaschen. Der unspezifische Anteil der Kinasereaktion (Blank) wird mit 100 nM Staurosporine bestimmt. Radioaktivität wird im Topcount gemessen. $IC_{50}$- Werte werden mit RS1 berechnet.

HPLC-Gradientensystem

Säule:

RP- select B (Merck KGaA, Cat. 1.050981)

Eluenten:

Eluent A: Wasser + 0.01 % TFA
Eluent B: Acetonitril + 0.01 % TFA
Flußrate: 1,5 ml/min
Injektionsvolumen: 10 μL

Gradient:

0 min 20 % B
6 min 100 % B

7 min 100 % B
8 min 20 % B
9 min 20 % B

Beispiel 1

Herstellung von 3-(1-Benzyl-1 H-[1,2,3]triazol-4-yl)-1H-pyrrolo[2,3-b]pyridin ("A1")

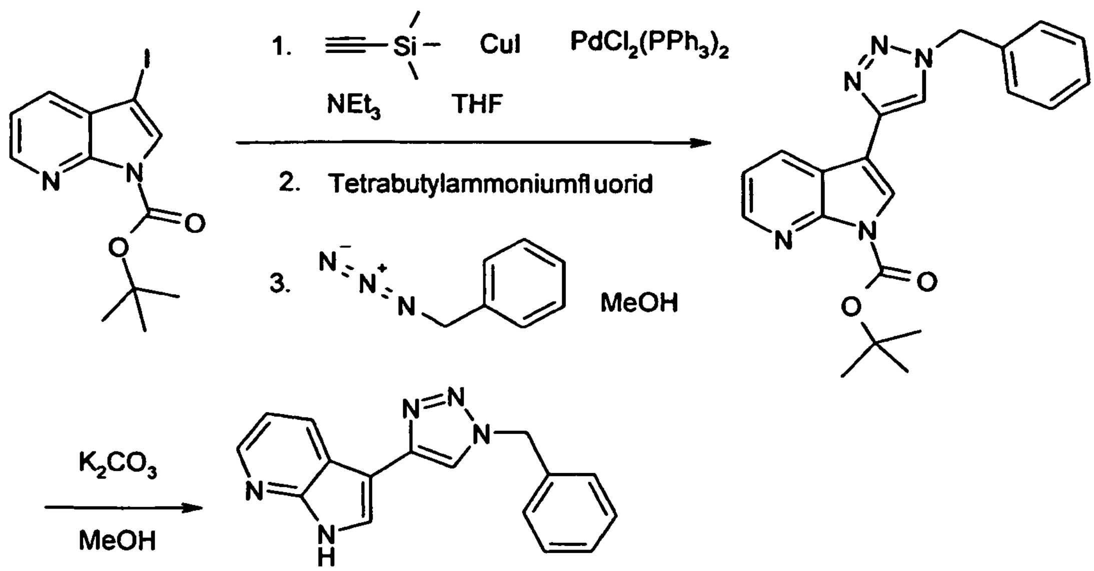


1.1 Zu einer unter Argon gehaltenen Suspension von 14 mg (0.02 mmol) Bis (triphenylphosphin) palladiumdichlorid, 8 mg (0.04 mmol) Kupfer (I) iodid und 344 mg (1.00 mmol) tert.- Butyl- 3- iod- (1 H)- pyrrolo [2, 3- b] pyridin- 1-carboxylat in 5 ml THF werden nacheinander 0.21 ml (1.50 mmol) Trimethylsilylacetylen und 0.28 ml (2.00 mmol) Triethylamin gegeben und die Mischung unter Argon eine Stunde bei Raumtemperatur gerührt. Dann wird zur Entfernung überschüssigen Trimethylsilylacetylens 5 Minuten Argon durch das Reaktionsgemisch durchgeleitet. Anschließend werden 1.10 ml (1.10 mmol) einer 1 M Lösung von Tetrabutylammoniumfluorid in THF zugegeben und das Reaktionsgemisch 30 Minuten bei Raumtemperatur gerührt. Danach wird eine Lösung von 136 mg (1.00 mmol) Benzylazid in 1 ml THF zugegeben und das Reaktionsgemisch 66 Stunden bei Raumtemperatur gerührt. Das Reaktionsgemisch wird auf Kieselgur adsorbiert und an einer Kieselgelsäule mit Petrolether/ Ethylacetat 2 : 1 chromatographiert. Man erhält 3- (1- Benzyl- 1 H- [1, 2, 3] triazol- 4- yl)- pyrrolo [2, 3- b] pyridin- 1- carbonsäure-tert- butylester als gelbes Öl; ESI 376.

1.2 Eine Lösung von 192 mg (0.51 mmol) 3- (1- Benzyl- 1H- [1, 2, 3] triazol- 4- yl)- pyrrolo [2, 3- b] pyridin- 1-carbonsäure- tert.- butylester in 2.6 ml Methanol wird mit 179 mg (1.28 mmol) Kaliumcarbonat versetzt und 1 Stunde bei Raumtemperatur gerührt. Das Reaktionsgemisch wird auf Kieselgur adsorbiert und an einer Kieselgelsäule mit Dichlormethan / Methanol / Ammoniakwasser chromatographiert. Man erhält 3- (1- Benzyl- 1 H- [1, 2, 3] triazol- 4-yl)- 1 H- pyrrolo [2, 3- b] pyridin als farblose Kristalle; ESI 276;
$^1$H- NMR ($d_6$- DMSO) : $\delta$ [ppm] = 5.66 (s, 2H), 7.17 (dd, $J_1$ = 7.9 Hz, $J_2$ = 4.7 Hz, 1H), 7.32- 7.43 (m, 5H), 7.92 (d, J = 2.5 Hz, 1 H), 8.29 (dd, $J_1$ = 4.7 Hz, $J_2$ =1.6 Hz, 1 H), 8.44 (dd, $J_1$ = 7.9 Hz, $J_2$ = 1.6 Hz, 1 H), 8.54 (s, 1 H), 11.9 (bs, 1 H) .

Beispiel 2

Herstellung von 3-[1-(4-Chlorbenzyl)-1H-[1,2,3]triazol-4-yl]-1H-pyrrolo[2,3-b]pyridin ("A2")

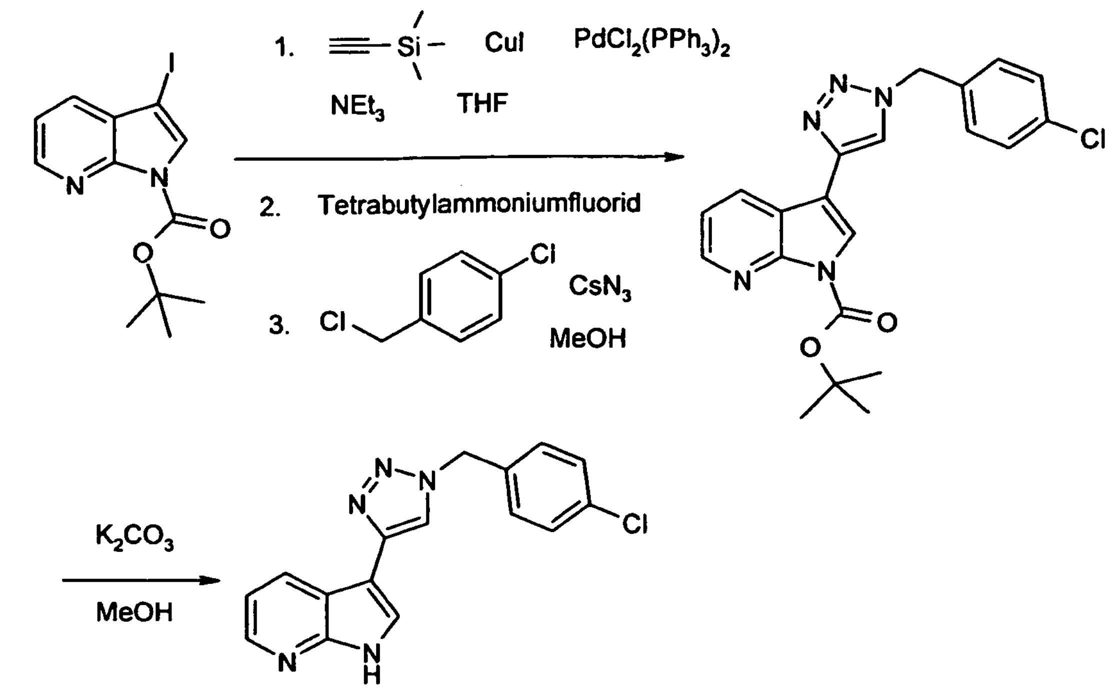


2.1 Zu einer unter Argon gehaltenen Suspension von 28 mg (0.04 mmol) Bis (triphenylphosphin) palladiumdichlorid, 16 mg (0.08 mmol) Kupfer (I) iodid und 688 mg (2.00 mmol) tert.- Butyl- 3- iod- (1 H)- pyrrolo [2, 3- b] pyridin- 1- carboxylat in 10 ml THF werden nacheinander 0.43 ml (3.00 mmol) Trimethylsilylacetylen und 0.55 ml (4.00 mmol) Triethylamin gegeben und die Mischung unter Argon eine Stunde bei Raumtemperatur gerührt. Dann wird zur Entfernung überschüssigen Trimethylsilylacetylens 5 Minuten Argon durch das Reaktionsgemisch durchgeleitet. Anschließend werden 2.00 ml (2.0 mmol) einer 1 M Lösung von Tetrabutylammoniumfluorid in THF zugegeben und das Reaktionsgemisch 30 Minuten bei Raumtemperatur gerührt. Danach wird eine Lösung von 322 mg (2.00 mmol) 1- Chlor- 4- (chlormethyl)- benzol in 5 ml Methanol, 350 mg (2.00 mmol) Caesiumazid und 5 ml Methanol zugegeben und das Reaktionsgemisch 51 Stunden bei Raumtemperatur gerührt. Das Reaktionsgemisch wird auf Kieselgur adsorbiert und an einer Kieselgelsäule mit Petrolether/ Ethylacetat 2 : 1 chromatographiert. Man erhält 3- [1- (4- Chlorbenzyl)- 1 H- [1, 2, 3] triazol- 4- yl]- pyrrolo [2, 3- b] pyridin- 1- carbonsäure- tert.- butylester als hellgelben Feststoff; ESI 410.

2.2 Eine Lösung von 347 mg (0.85 mmol) 3- [1- (4- Chlorbenzyl)- 1 H- [1, 2, 3] triazol- 4- yl]- pyrrolo [2, 3- b] pyridin- 1- carbonsäure- tert.- butylester in 4.2 ml Methanol wird mit 295 mg (2.13 mmol) Kaliumcarbonat versetzt und 1 Stunde bei Raumtemperatur gerührt. Das Reaktionsgemisch wird auf Kieselgur adsorbiert und an einer Kieselgelsäule mit Dichlormethan / Methanol / Ammoniakwasser chromatographiert. Man erhält 3- [1- (4- Chlorobenzyl)- 1 H- [1, 2, 3] triazol- 4- yl]- 1H- pyrrolo [2, 3- b] pyridin als farblose Kristalle; ESI 310; $^1$H- NMR ($d_6$- DMSO) : $\delta$ [ppm] = 5.67 (s, 2H), 7.18 (dd, $J_1$ = 7.9 Hz, $J_2$ = 4.7 Hz, 1 H), 7.38- 7.43 (m, 2H), 7.45- 7.50 (m, 2H), 7.92 (d, J = 2.2 Hz, 1 H), 8.29 (d, J = 4.4 Hz, 1 H), 8.4 (d, J = 7.9 Hz, 1 H), 8.53 (s, 1H), 11.9 (bs, 1 H) .

Analog wird die nachstehende Verbindung erhalten

| Verbindung Nr. | Name und/oder Struktur | |
|---|---|---|
| "A3" | 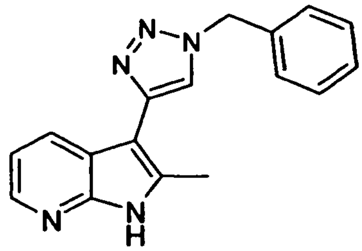 3-(1-Benzyl-1H-[1,2,3]triazol-4-yl)-2-methyl-1H-pyrrolo[2,3-b]pyridin | |

Beispiel 3

Herstellung von 3-(1-Benzyl-1H-[1,2,3]triazol-4-yl)-5-(1-methyl-1H-pyrazol-4-yl)-1 H-pyrrolo[2,3-b]pyridin ("A4")

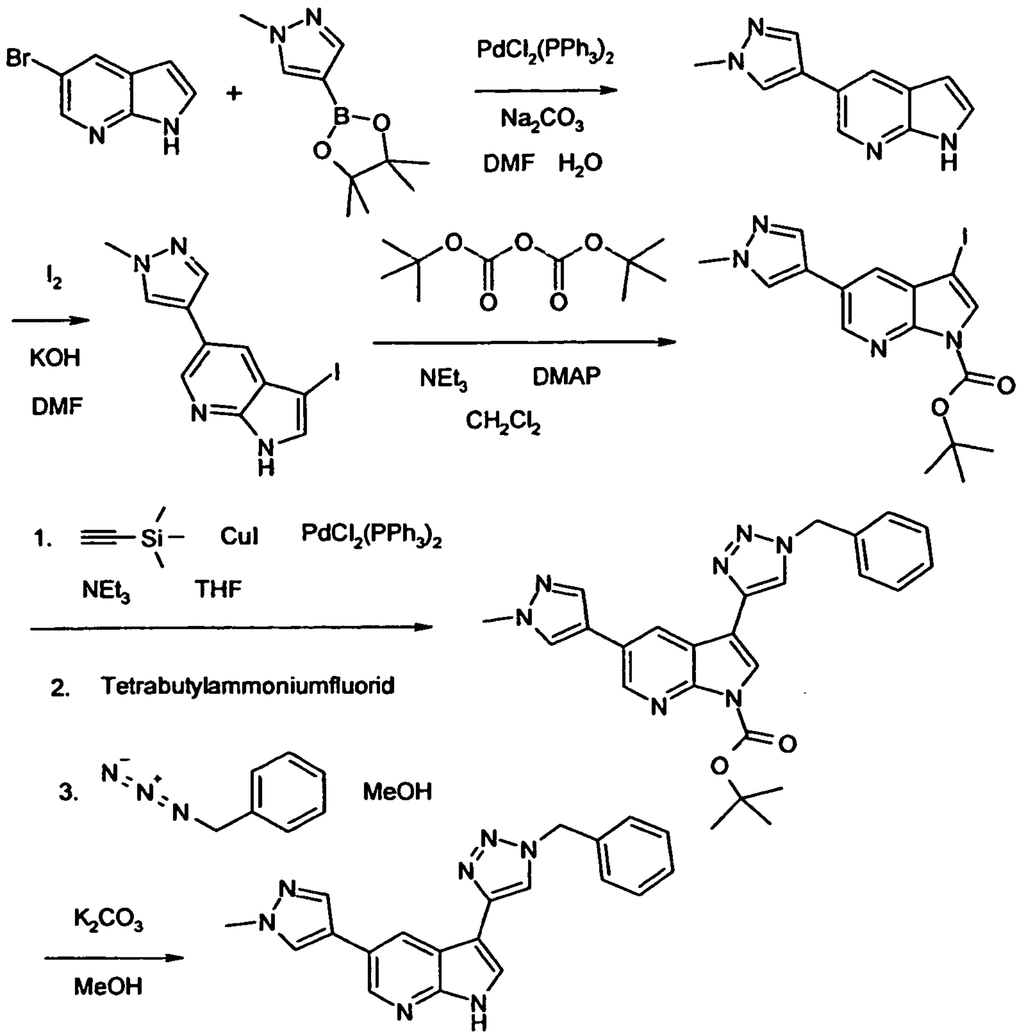


3.1 Eine unter Stickstoff gehaltene Lösung von 5.00 g (25.4 mmol) 5- Brom- 7- azaindol und 9.00 g (43.3 mmol) 1- Methyl- 1H- pyrazol- 4- boronsäure- pinacolester in 100 ml DMF wird mit 38 ml (76 mmol) einer 2 N Natriumcarbonat-Lösung und 1.48 g (1.28 mmol) Tetrakis (triphenylphosphin)- palladium versetzt und 1 Stunde bei 100° C gerührt. Das Reaktionsgemisch wird auf Raumtemperatur gekühlt und zwischen Wasser und Ethylacetat verteilt. Die organische Phase wird über Natriumsulfat getrocknet und eingedampft. Der Rückstand wird aus tert.- Butylmethylether kristallisiert: 5- (1- Methyl- 1 H- pyrazol- 4- yl)- 1 H- pyrrolo [2, 3- b] pyridin als gelblicher Feststoff; ESI199.

3.2 Zu einer Lösung von 4.00 g (20.2 mmol) 5- (1- Methyl- 1H- pyrazol- 4- yl)- 1 H- pyrrolo [2, 3- b] pyridin in 60 ml DMF werden 2.80 g (49.9 mmol) festes Kaliumhydroxid gegeben und dann unter Rühren eine Lösung von 5.10 g (20.1 mmol) Iod in 40 ml DMF langsam zugetropft. Das Reaktionsgemisch wird mit Wasser und 300 mg Natriumdisulfit versetzt und mit Ethylacetat extrahiert. Die organische Phase wird über Natriumsulfat getrocknet und eingedampft: 3- Iod- 5- (1- methyl- 1H- pyrazol- 4- yl)- 1H- pyrrolo [2, 3- b] pyridin als gelbliche Kristalle; ESI 325.

3.3 Zu einer Suspension von 5.85 g (18.0 mmol) 3- Iod- 5- (1- methyl- 1 H- pyrazol- 4- yl)- 1 H- pyrrolo [2, 3- b] pyridin in 100 ml Dichlormethan werden 7.5 ml (54.1 mmol) Triethylamin und 220 mg (1.80 mmol) 4- (Dimethylamino)-pyridin gegeben. Dann wird langsam eine Lösung von 4.6 ml (21.5 mmol) Di- tert- butyldicarbonat in 50 ml Dichlormethan zugetropft. Nach 4 Stunden Rühren bei Raumtemperatur wird das Reaktionsgemisch zwischen Wasser und Dichlormethan verteilt. Die organische Phase wird über Natriumsulfat getrocknet und im Vakuum eingedampft:

3- Iod- 5- (1- methyl- 1H- pyrazol- 4- yl)- pyrrolo [2, 3- b] pyridin- 1- carbonsäure- tert- butylester als farblose Kristalle; ESI 425.

3.4 Zu einer unter Argon gehaltenen Suspension von 6 mg (0.009 mmol) Bis (triphenylphosphin) palladiumdichlorid, 3 mg (0.016 mmol) Kupfer (I) iodid und 178 mg (0.42 mmol) 3- Iod- 5- (1- methyl- 1H- pyrazol- 4- yl)- pyrrolo [2, 3- b] pyridin- 1- carbonsäure- tert.- butylester in 2 ml THF werden nacheinander 0.09 ml (0.63 mmol) Trimethylsilylacetylen und 0.12 ml (0.84 mmol) Triethylamin gegeben und die Mischung unter Argon eine Stunde bei Raumtemperatur gerührt. Dann wird zur Entfernung überschüssigen Trimethylsilylacetylens 5 Minuten Argon durch das Reaktionsgemisch durchgeleitet. Anschließend werden 0.45 ml (0.45 mmol) einer 1 M Lösung von Tetrabutylammoniumfluorid in THF zugegeben und das Reaktionsgemisch 30 Minuten bei Raumtemperatur gerührt. Danach wird eine Lösung von 58 mg (0.42 mmol) Benzylazid in 2 ml Methanol zugegeben und das Reaktionsgemisch 48 Stunden bei Raumtemperatur gerührt. Das Reaktionsgemisch wird auf Kieselgur adsorbiert und an einer Kieselgelsäule mit Petroether/ Ethylacetat als Laufmittel chromatographiert. Man erhält 3- (1- Benzyl- 1 H- [1, 2, 3] triazol- 4- yl)- 5- (1- methyl- 1H- pyrazol- 4- yl)- pyrrolo [2, 3- b] pyridin- 1- carbonsäure- tert.- butylester als farblos Kristalle; ESI 456.

3.5 Eine Lösung von 115 mg (0.25 mmol) 3- (1- Benzyl- 1H- [1, 2, 3] triazol- 4- yl)- 5- (1- methyl- 1H- pyrazol- 4- yl)- pyrrolo [2, 3- b] pyridin- 1- carbonsäure- tert- butylester in 1.3 ml Methanol wird mit 88 mg (0.63 mmol) Kaliumcarbonat versetzt und 1 Stunde bei Raumtemperatur gerührt. Das Reaktionsgemisch wird auf Kieselgur adsorbiert und an einer Kieselgelsäule mit Dichlormethan/ Methanol/ Ammoniakwasser chromatographiert. Man erhält 3- (1- Benzyl- 1 H- [1, 2, 3] triazol- 4- yl)- 5- (1- methyl- 1H- pyrazol- 4- yl)- 1 H- pyrrolo [2, 3- b] pyridin als farblose Kristalle; ESI 356;
$^1$H- NMR ($d_6$- DMSO) : $\delta$ [ppm] = 3.9 (s, 3H), 5.68 (s, 2H), 7.33- 7.43 (m, 5H), 7.92 (d, J = 2.8 Hz, 1 H), 7.95- 7.96 (m, 1 H), 8.21 (s, 1 H), 8.50 (d, J = 2.2 Hz, 1 H), 8.54 (d, J =2.2 Hz, 1 H), 8.62 (s, 1H), 11.88 (bs, 1 H) .

Analog werden die nachstehenden Verbindungen erhalten

| Verbindung Nr. | Name und/oder Struktur | HPLC/MS [M+H]+ |
|---|---|---|
| "A5" | 3-[1-(3-Fluor-benzyl)-1H-[1,2,3]triazol-4-yl]-5-(1-methyl-1H-pyrazol-4-yl)-1H-pyrrolo[2,3-b]pyridin 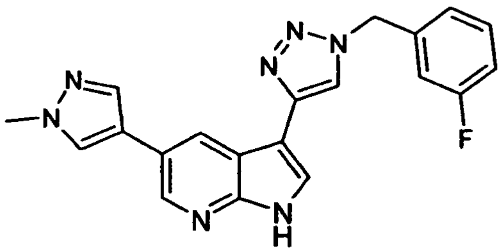 | Rt = 1.819 min / 374 |
| "A6" | 3-[1-(4-Fluor-benzyl)-1H-[1,2,3]triazol-4-yl]-5-(1-methyl-1H-pyrazol-4-yl)-1H-pyrrolo[2,3-b]pyridin 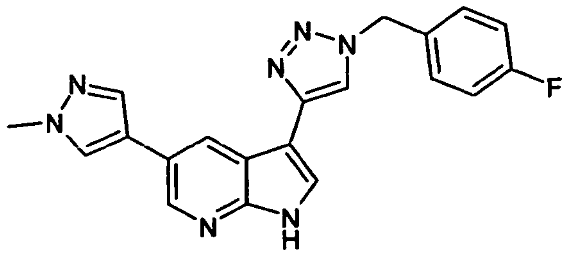 | Rt = 1.821 min / 374 |
| "A7" | 3-[1-(3-Methyl-benzyl)-1H-[1,2,3]triazol-4-yl]-5-(1-methyl-1H-pyrazol-4-yl)-1H-pyrrolo[2,3-b]pyridin | Rt = 1.890 min / 370 |

(fortgesetzt)

| Verbindung Nr. | Name und/oder Struktur | HPLC/MS [M+H]+ |
|---|---|---|
| | 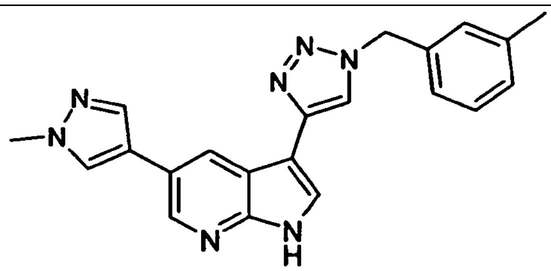 | |
| | | |
| "A9" | 5-(1-Methyl-1H-pyrazol-4-yl)-3-(1-pyridin-4- ylmethyl-1H-[1,2,3]triazol-4-yl)-1 H-pyrrolo[2,3-b]pyridin 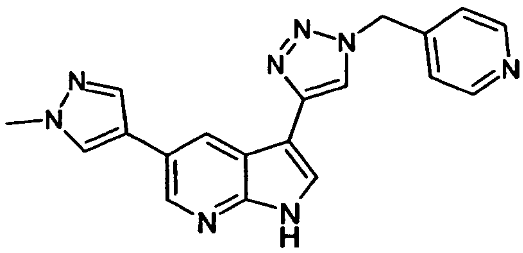 | Rt = 1.215 min / 357 |
| Die 4. Stufe wird analog zu Beispiel 2 mit 4-Chlormethylpyridin und Caesiumazid durchgeführt. | | |
| "A10" | 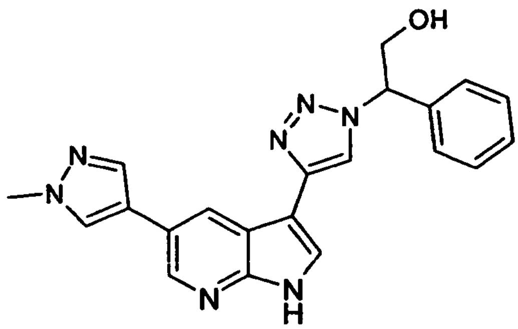 2-{4-[5-(1-Methyl-1H-pyrazol-4-yl)-1H-pyrrolo[2,3-b] pyridin-3-yl]-[1,2,3]triazol-1-yl}-2-phenyl-ethanol | |
| Die 4. Stufe wird analog zu Beispiel 2 mit 1-Phenyl-2-(tert.butyldimethyl-silanyloxy)-ethyl-methansulfonat (Herstellung analog zu H. Kotsuki et al. J. Org. Chem. 61, 1996, S. 984) und Caesiumazid durchgeführt. | | |
| "A11" | 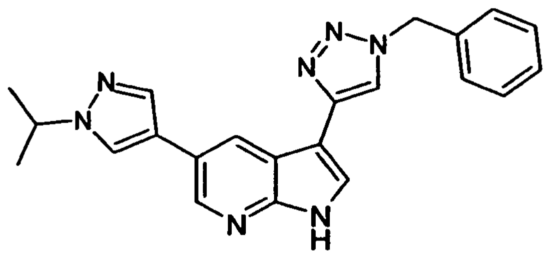 | |
| "A12" | 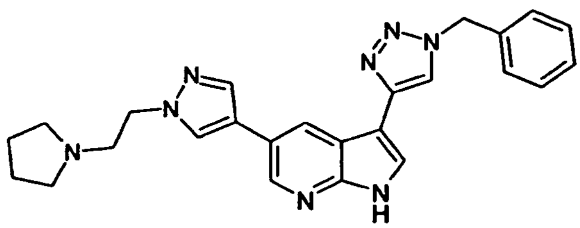 | |

(fortgesetzt)

| Verbindung Nr. | Name und/oder Struktur | HPLC/MS [M+H]+ |
|---|---|---|
| "A13" | 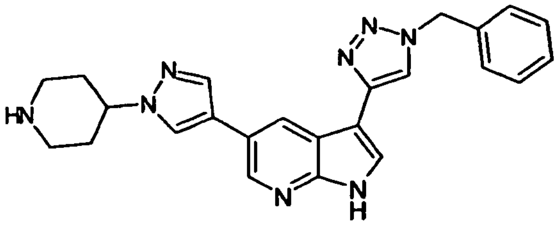 | |
| Bei der Herstellung wird Boc-geschütztes Piperidinderivat verwendet; als letzte Stufe wird mit Dioxan/HCl die Schutzgruppe entfernt. | | |

Beispiel 4

Herstellung von 2-{4-[3-(1-Benzyl-1H-[1,2,3]triazol-4-yl)-1 H-pyrrolo[2,3-b]pyridin-5-yl]-pyrazol-1-yl}-ethanol ("A14")

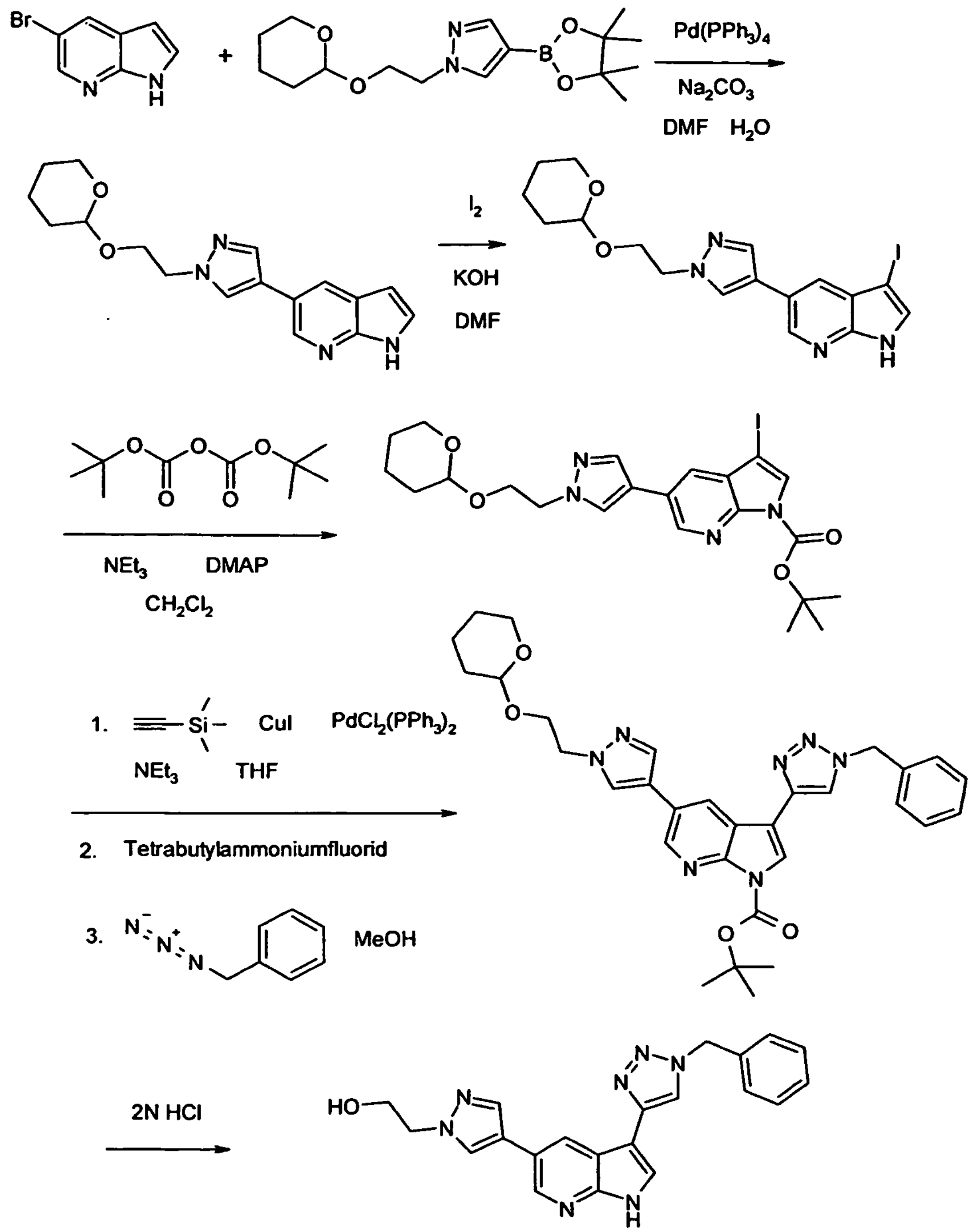


Beispiel 5

Herstellung von 3-(1-Benzyl-1H-[1,2,3]triazol-4-yl)-4-methoxy-1H-pyrrolo[2,3-b]pyridin("A15")

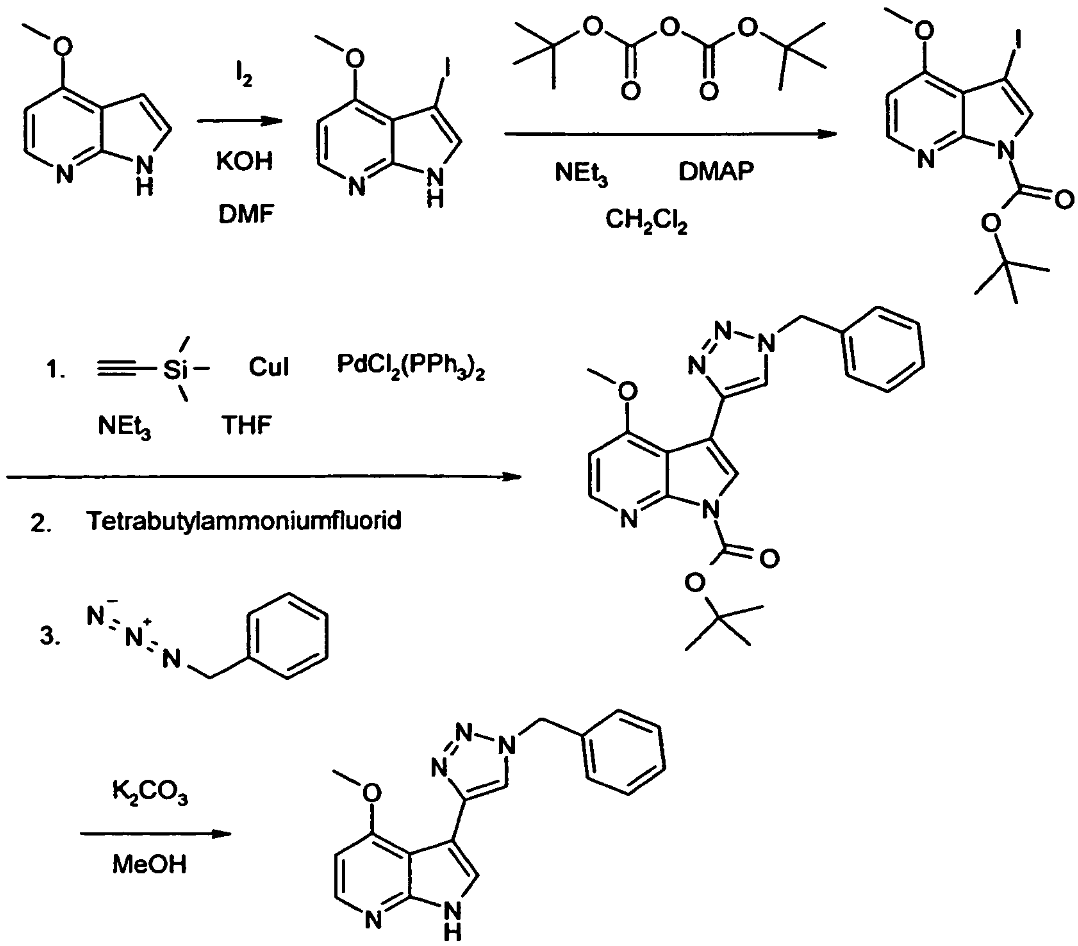


Analog der obenstehenden Beispiele werden die nachstehenden Verbindungen erhalten

| Verbindung Nr. | Name und/oder Struktur | HPLC/MS [M+H]+ |
|---|---|---|
| "A16" | 5-(1-Methyl-1H-pyrazol-4-yl)-3-(1-phenethyl-1H-[1,2,3]triazol-4-yl)-1H-pyrrolo[2,3-b]pyridin 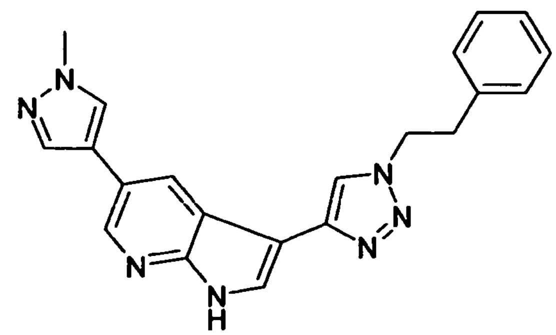 | Rt = 1.828 min / 370 |
| $^1$H-NMR [DMSO-$d_6$]: δ [ppm] = 11.81, 8.52 (d, *J= 1.72 Hz,* 1H), 8.47 (s, 1H), 8.41 (d, *J= 1.42 Hz*, 1H), 8.18 (s, 1H), 7.93 (s, 1H), 7.84 (d, *J= 2.36 Hz,* 1H), 7.31-7.20 (m, 5H), 4.68 (t, *J= 7.31 Hz,* 2H), 3.90 (s, 3H), 3.27 (t, *J= 7.29 Hz,* 2H) | | |
| "A17" | 3-[1-(2-Fluor-benzyl)-1H-[1,2,3]triazol-4-yl]-5-(1-methyl-1H-pyrazol-4-yl)-1H-pyrrolo[2,3-b]pyridin | Rt = 1.799 min / 374 |

(fortgesetzt)

| Verbindung Nr. | Name und/oder Struktur | HPLC/MS [M+H]+ |
|---|---|---|
| | 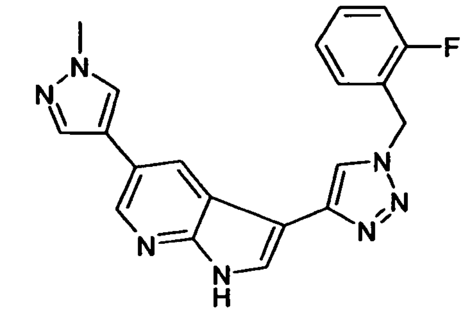 | |
| $^1$H-NMR [DMSO-d$_6$]: δ [ppm] = 11.86 (s, 1H), 8.57 (s, 1H), 8.53 (d, *J=2 Hz,* 1H), 8.48 (d, *J=2 Hz,* 1H), 8.19 (s, 1H), 7.93 (s, 1H), 7.90 (d, *J=2.6 Hz,* 1H), 7.46-7.42 (m, 1H), 7.38 (m, 1H), 7.27 (m, 2H), 5.73 (s, 2H), 3.90 (s, 3H) | | |
| "A18" | 5-(1-Methyl-1H-pyrazol-4-yl)-3-[1-(2-morpholin-4-yl-ethyl)-1H-[1,2,3]triazol-4-yl]-1H-pyrrolo[2,3-b]pyridin 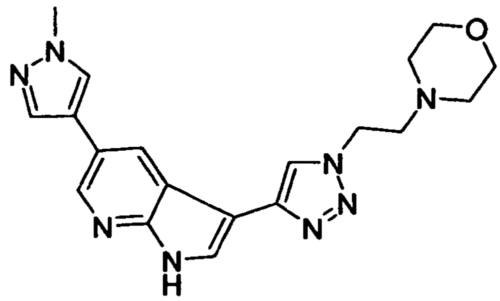 | Rt = 1.078 min / 379 |
| "A19" | 5-(1-Methyl-1H-pyrazol-4-yl)-3-{1-[1-(3-trifluormethyl-phenyl)-ethyl]-1H-[1,2,3]triazol-4-yl}-1H-pyrrolo[2,3-b]pyridin 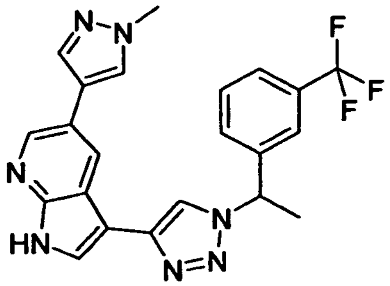 | Rt = 2.064 min / 438 |
| $^1$H-NMR [DMSO-d$_6$]: δ [ppm] = 11.82 (s, NH), 8.70 (s, 1H), 8.51 (d, *J=2.05 Hz,* 1H), 8.49 (d, *J=2.03 Hz,* 1H), 8.19 (s, 1H), 7.93 (d, *J=0.73 Hz,* 1H), 7.89 (d, *J=2.56 Hz,* 1H), 7.96-7.63 (m, 4 H), 6.16 (q, *J=7.02 Hz,* 1H), 3.90 (s, 3H), 2.02 (d, *J=7.10 Hz,* 3H) | | |
| "A20" | 5-(1-Methyl-1H-pyrazol-4-yl)-3-{1-[1-(2-trifluormethyl-phenyl)-ethyl]-1H-[1,2,3]triazol-4-yl}-1H-pyrrolo[2,3-b]pyridin | Rt = 2.085 min / 438 |

(fortgesetzt)

| Verbindung Nr. | Name und/oder Struktur | HPLC/MS [M+H]+ |
|---|---|---|
| | 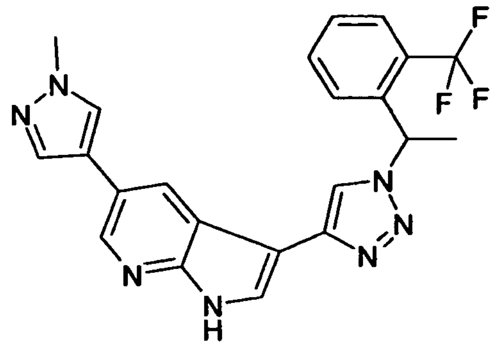 | |
| "A21" | 3-[1-(2,4-Difluor-benzyl)-1H-[1,2,3]triazol-4-yl]-5-(1-methyl-1H-pyrazol-4-yl)-1H-pyrrolo[2,3-b]pyridin 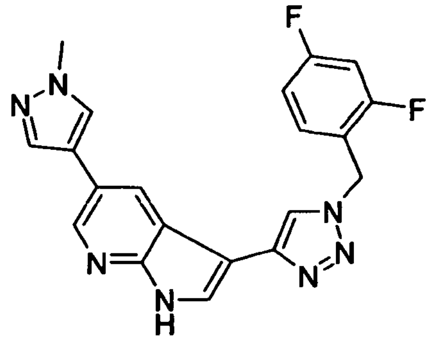 | Rt = 1.847 min / 392 |
| $^1$H-NMR [DMSO-$d_6$]: δ [ppm] =11.83 (s, NH), 8.55 (s, 1H), 8.52 (d, *J= 1.95 Hz,* 1H), 8.47 (d, J= *1.78 Hz,* 1 H), 8.18 (s, 1H), 7.91 (s, 1H), 7.89 (d, J= *2.52 Hz,* 1H), 7.49 (dd, *J= 8.55 Hz, J=15.22 Hz,* 1H), 7.33 (m, 1 H), 7.15 (t, *J= 7.43 Hz,* 1H), 5.70 (s, 2H), 3.89 (s, 3H) | | |
| "A22" | 3-[1-(2-Fluor-3-trifluormethyl-benzyl)-1H-[1,2,3]triazol-4-yl]-5-(1-methyl-1H-pyrazol-4-yl)-1H-pyrrolo[2,3-b]pyridin 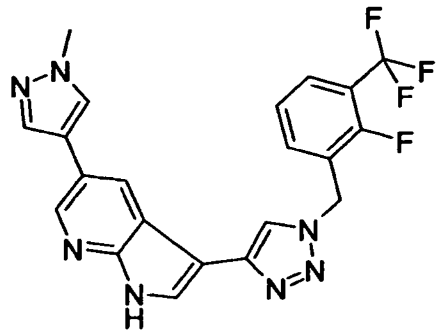 | Rt = 2.001 min / 442 |
| "A23" | 3-[1-(3-Chlor-2-fluor-benzyl)-1H-[1,2,3]triazol-4-yl]-5-(1-methyl-1H-pyrazol-4-yl)-1H-pyrrolo[2,3-b]pyridin 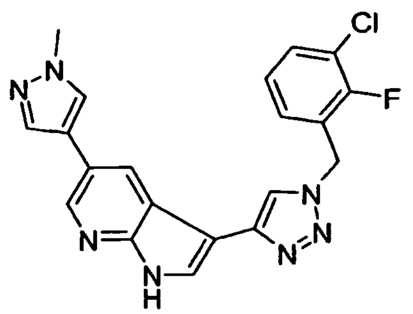 | Rt = 1.949 min / 408 |

(fortgesetzt)

| Verbindung Nr. | Name und/oder Struktur | HPLC/MS [M+H]+ |
|---|---|---|
| $^1$H-NMR [DMSO-d$_6$]: δ [ppm] = 11.84 (s, NH), 8.59 (s, 1H), 8.52 (d, *J= 2.00* | | |
| *Hz*, 1H), 8.47 (d, *J= 2.02 Hz,* 1H), 8.18 (s, 1H), 7.91 (s, 1H), 7.90 (s, 1H), 7.61 (t, *J= 6.81 Hz,* 1H), 7.34 (t, *J= 6.45 Hz,* 1H), 7.27 (t, *J= 7.87 Hz,* 1H), 5.78 (s, 2H), 3.89 (s, 3H) | | |
| "A24" | 3-[1-(2-Fluor-6-trifluormethyl-benzyl)-1H-[1,2,3]triazol-4-yl]-5-(1-methyl-1H-pyrazol-4- yl)-1H-pyrrolo[2,3-b]pyridin 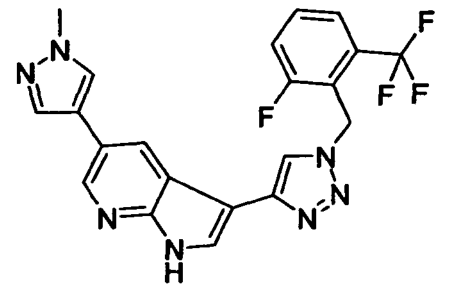 | Rt = 1.940 min / 442 |
| "A25" | 3-[1-(2,3-Difluor-benzyl)-1H-[1,2,3]triazol-4- yl]-5-(1-methyl-1H-pyrazol-4-yl)-1H-pyrrolo[2,3-b]pyridin 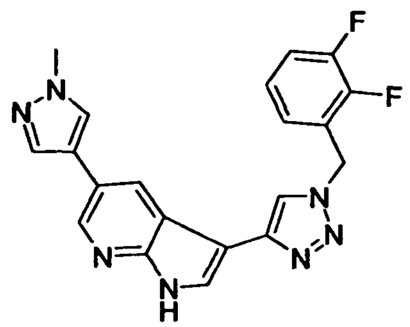 | Rt = 1.843 min / 392 |
| $^1$H-NMR [DMSO-d$_6$]: δ [ppm] = 11.85 (s, NH), 8.59 (s, 1H), 8.52 (d, *J*= 2.04 *Hz,* 1H), 8.48 (d, *J= 1.98 Hz,* 1H), 8.19 (s, 1H), 7.92 (s, 1H), 7.90 (d, *J= 2.63 Hz,* 1H), 7.46 (dd, J= 8.33 Hz, *J=16.99 Hz,* 1H), 7.26 (dd, *J=8.05 Hz, J=12.92 Hz,* 1H), 7.19 (m, 1H), 5.79 (s, 2H), 3.89 (s, 3H) | | |
| "A26" | 3-[1-(2-Fluor-4-trifluormethyl-benzyl)-1H-[1,2,3]triazol-4-yl]-5-(1-methyl-1H-pyrazol-4-yl)-1H-pyrrolo[2,3-b]pyridin | Rt = 2.036 min / 442 |
| | 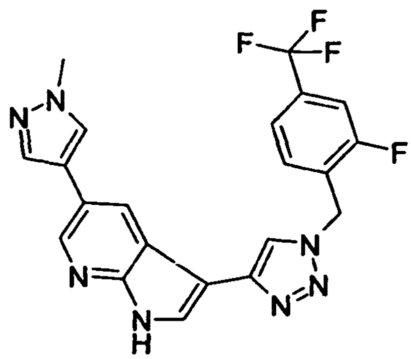 | |
| "A27" | 4-(1-{4-[5-(1-Methyl-1H-pyrazol-4-yl)-1H-pyrrolo[2,3-b] pyridin-3-yl]-[1,2,3]triazol-1-yl}-ethyl)-benzoesäure | Rt = 1.699 min / 414 |

(fortgesetzt)

| Verbindung Nr. | Name und/oder Struktur | HPLC/MS [M+H]+ |
|---|---|---|
| | 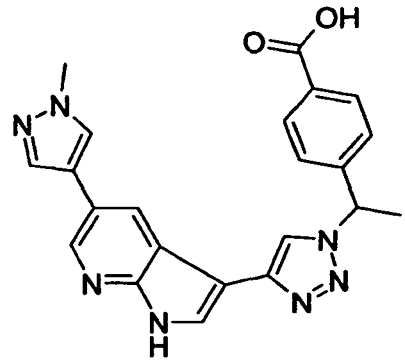 | |
| $^1$H-NMR [DMSO-d$_6$]: δ [ppm] = 12.95 (br, 1H), 11.83 (s, 1H), 8.64 (s, 1H), 8.51 (d, *J= 1.99 Hz,* 1H), 8.49 (d, *J= 1.89 Hz,* 1H), 8.18 (s, 1H), 7.96-7.88 (m, 4H), 7.46 (s, 1H), 7.45 (s, 1H), 6.10 (q, *J= 6.86* Hz, 1H), 3.89 (s, 3H), 1.99 (d, *J=5 Hz,* 3H) | | |
| "A28" | 3-{4-[5-(1-Methyl-1H-pyrazol-4-yl)-1H-pyrrolo[2,3-b]pyridin-3-yl]-[1,2,3]triazol-1-yl}-3-phenyl-propionsäure 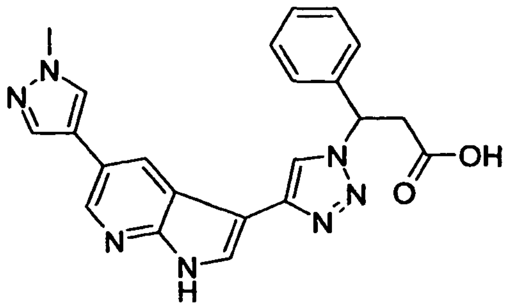 | Rt = 1.661 min / 414 min |
| "A29" | 6-{4-[5-(1-Methyl-1H-pyrazol-4-yl)-1H-pyrrolo[2,3-b]pyridin-3-yl]-[1,2,3]triazol-1-ylmethyl}-chinolin 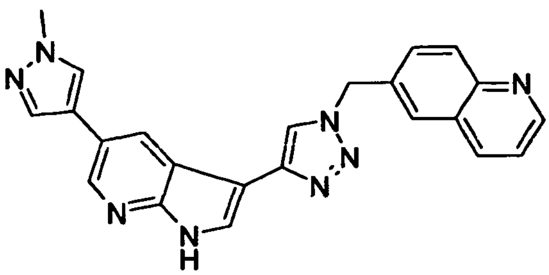 | Rt = 1.421 min / 407 min |
| $^1$H-NMR [DMSO-d$_6$]: δ [ppm] = 11.83 (s, 1H), 8.91 (dd, *J=1.65 Hz, J=4.18 Hz,* 1H), 8.65 (s, 1H), 8.52 (d, *J= 2.03 Hz,* 1H), 8.48 (d, *J=1.97 Hz,* 1H), 8.39 (d, *J=7.89 Hz,* 1H), 8.18 (s, 1H), 8.05 (d, *J= 8.71 Hz,* 1H), 7.94 (s, 1H), 7.92 (s, 1H), 7.89 (d, *J=2.60 Hz,* 1H), 7.75 (dd, *J=1.91 Hz, J=8.74 Hz*, 1H), 7.55 (dd, *J= 4.20 Hz, J=8.34 Hz,* 1H), 5.89 (s, 2H), 3.89 (s, 3H) | | |
| "A30" | [4-(1-{4-[5-(1-Methyl-1H-pyrazol-4-yl)-1H-pyrrolo[2,3-b] pyridin-3-yl]-[1,2,3]triazol-1-yl}-ethyl)-phenyl]-methanol 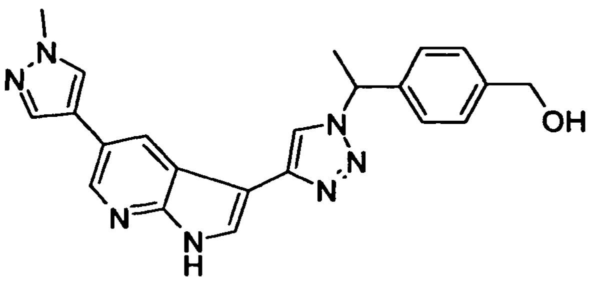 | Rt = 1.602 min / 400 |

(fortgesetzt)

| Verbindung Nr. | Name und/oder Struktur | HPLC/MS $[M+H]^+$ |
|---|---|---|
| | Erhältlich durch Reduktion von "A27" mit $LiAlH_4$. | |
| "A31" | 2-{4-[5-(1-Methyl-1H-pyrazol-4-yl)-1H-pyrrolo[2,3-b]pyridin-3-yl]-[1,2,3]triazol-1-yl}-1-phenyl-ethanol 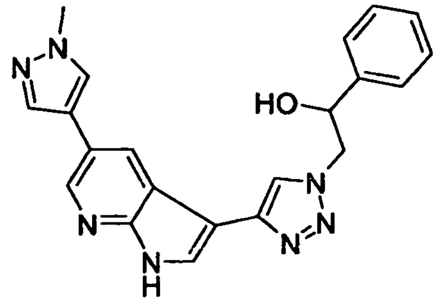 | Rt = 1.636 min / 386 |
| $^1$H-NMR [DMSO-$d_6$]: δ [ppm] = 11.84 (s, 1H), 8.53 (d, *J=2.06 Hz,* 1H), 8.49 (s, 1H), 8.45 (d, *J= 2.03 Hz,* 1H), 8.21 (s, 1H), 7.94 (s, 1H), 7.89 (d, *J=2.52 Hz,* 1H), 7.45-7.28 (m, 5 H), 5.88 (d, *J= 4.65 Hz,* 1H), 5.09 (m, 1H), 4.56 (ddd, *J=6.39 Hz, J=13.81 Hz, J=22.25 Hz,* 2H), 3.90 (s, 3H) | | |
| "A32" | 3-[1-(3-Fluor-pyridin-4-ylmethyl)-1H-[1,2,3]triazol-4-yl]-5-(1-methyl-1H-pyrazol-4-yl)-1H-pyrrolo[2,3-b]pyridin 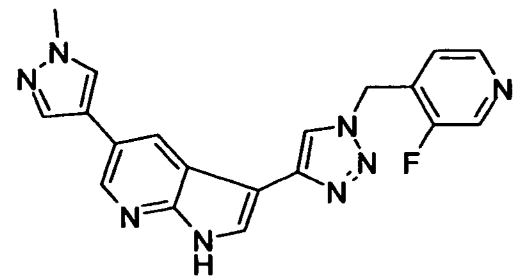 | Rt = 1.523 min / 375 |
| $^1$H-NMR [DMSO-$d_6$]: δ [ppm] = 11.89 (s, 1H), 8.65 (s, 2H), 8.53 (d, *J= 2.06 Hz*, 1H), 8.48 (m, 2H), 8.20 (s, 1H), 7.93 (m, 2H), 7.24 (m, 1H), 5.85 (s, 2H), 3.89 (s, 3H) | | |
| "A33" | 3-[1-(2-Chlor-benzyl)-1H-[1,2,3]triazol-4-yl]-5-(1-methyl-1H-pyrazol-4-yl)-1H-pyrrolo[2,3-b]pyridin 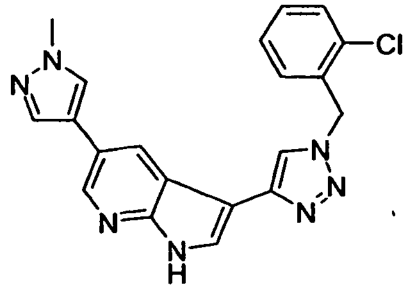 | Rt = 1.857 min / 390 |
| "A34" | 3-[1-(3-Methoxy-benzyl)-1H-[1,2,3]triazol-4-yl]-5-(1-methyl-1H-pyrazol-4-yl)-1H-pyrrolo[2,3-b]pyridin 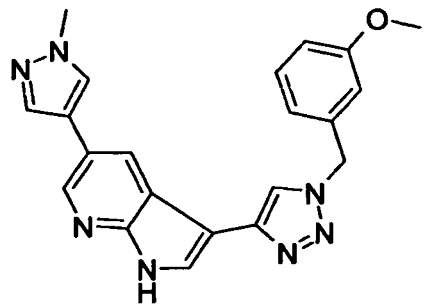 | Rt = 1.769 min / 386 |

(fortgesetzt)

| Verbindung Nr. | Name und/oder Struktur | HPLC/MS [M+H]+ |
|---|---|---|
| "A35" | (2-{4-[5-(1-Methyl-1H-pyrazol-4-yl)-1H-pyrrolo[2,3-b] pyridin-3-yl]-[1,2,3]triazol-1-ylmethyl}-phenyl)-methanol 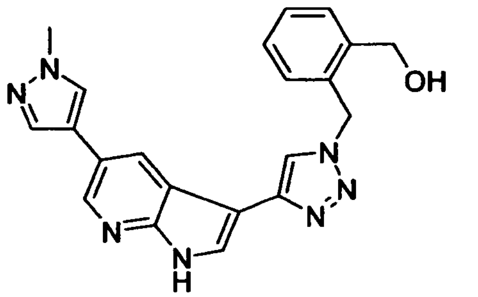 | Rt = 1.536 min / 386 |
| $^1$H-NMR [DMSO-$d_6$]: δ [ppm] = 11.82 (s, 1H), 8.52 (s, 2H), 8.48 (d, *J=1.99 Hz,* 1H), 8.18 (s, 1H), 7.91 (s, 1H), 7.88 (d, *J=2.59 Hz,* 1H), 7.46 (d, *J=7.48 Hz*, 1H), 7.33 (t, *J=7.41 Hz,* 1H), 7.27 (t, *J= 7.07 Hz,* 1H), 7.08 (d, *J= 7.48 Hz,* 1H), 5.74 (s, 2H), 5.29 (t, *J= 5.37 Hz,* 1H), 4.68 (d, *J= 5.37 Hz,* 2H), 3.89 (s, 3H) | | |
| "A36" | 3-[1-(4-Difluormethoxy-benzyl)-1H-[1,2,3]triazol-4-yl]-5-(1-methyl-1H-pyrazol-4-yl)-1H-pyrrolo[2,3-b]pyridin 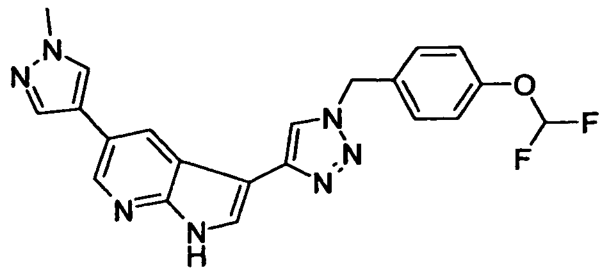 | Rt = 1.877 min / 422 |
| "A37" | 3-{4-[5-(1-Methyl-1H-pyrazol-4-yl)-1H-pyrrolo[2,3-b]pyridin-3-yl]-[1,2,3]triazol-1-ylmethyl}-benzoesäure-methylester 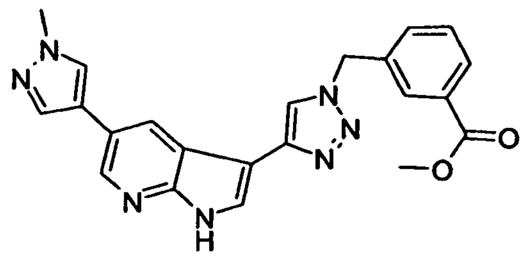 | Rt = 1.749 min / 414 |
| "A38" | 2-{4-[5-(1-Methyl-1H-pyrazol-4-yl)-1H-pyrrolo[2,3-b]pyridin-3-yl]-[1,2,3]triazol-1-ylmethyl}benzonitril 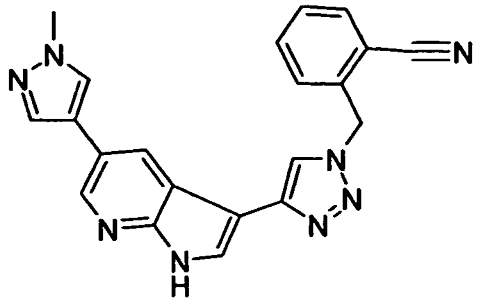 | Rt = 1.680 min / 381 |
| $^1$H-NMR [DMSO-$d_6$]: δ [ppm] = 11.85 (s, 1H), 8.62 (s, 1H), 8.53 (d, *J= 2.03 Hz,* 1H), 8.47 (d, *J=1.94 Hz,* 1H), 8.18 (s, 1H), 7.94 (d, *J= 7.72 Hz,* 1H), 7.91 (m, 2H), 7.75 (t, *J= 7.74 Hz,* 1H), 7.58 (t, *J=7.24 Hz,* 1H), 7.43 (d, *J= 7.81 Hz,* 1H), 5.89 (s, 2H), 3.89 (s, 3H) | | |

(fortgesetzt)

| Verbindung Nr. | Name und/oder Struktur | HPLC/MS $[M+H]^+$ |
|---|---|---|
| "A39" | 3-[1-(3-Difluormethoxy-benzyl)-1H-[1,2,3]triazol-4-yl]-5-(1-methyl-1H-pyrazol-4- yl)-1H-pyrrolo[2,3-b]pyridin 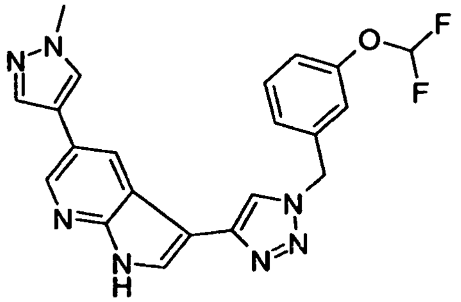 | Rt = 1.876 min / 422 |
| "A40" | 3-{4-[5-(1-Methyl-1H-pyrazol-4-yl)-1H-pyrrolo[2,3-b]pyridin-3-yl]-[1,2,3-triazol-1-yl}-3-phenyl-propan-1-ol 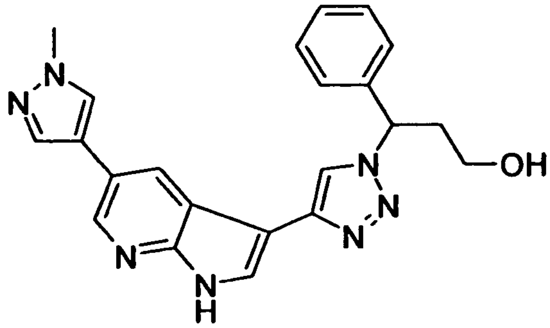 Erhältlich durch Reduktion von "A28" mit $LiAlH_4$ | |

Beispiel 6

Herstellung von 5-(1-Methyl-1H-pyrazol-4-yl)-3-[1-(1-phenyl-ethyl)-1H-[1,2,3]triazol-4-yl]-1H-pyrrolo[2,3-b]pyridin ("A8")

6.1

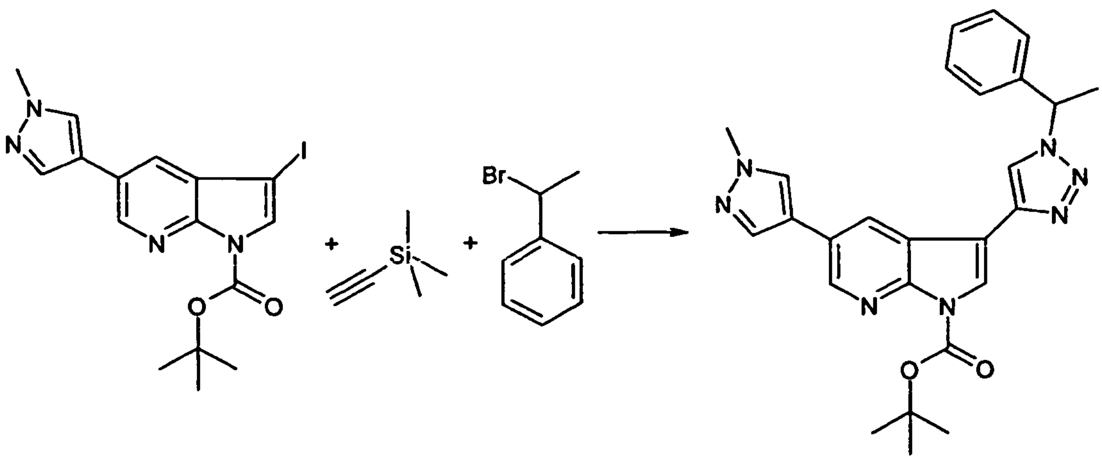

Pd $(PPh_3)_2Cl_2$ (12 mg, 0.017 mmol) und CuI (7 mg, 0.037 mmol) werden vorgelegt, anschließend wird eine Lösung von 3- Iodo- 5- (1- methyl- 1H- pyrazol- 4- yl)- pyrrolo [2, 3- b] pyridin- 1- carbonsäure- *tert*- butylester (350 mg, 0.825 mmol) in 5 ml THF zugegeben und mit Stickstoff inertisiert. Zum Schluß gibt man TMS- Acetylen (0.175 ml, 1.263

mmol) und Triethylamin (0.230 ml, 1.659 mmol) zu und rührt ca. 1, 5h bei RT unter Stickstoff- Schutz. Das überschüssige TMS- Acetylen wird entfernt, indem man das dunkle Reaktionsgemisch ca. 10 min. intensiv mit Stickstoff gespült. Danach wird die TBAF- Lösung (1 ml, 0.990 mmol, 1 M in THF) zugegeben und 0.5 h gerührt.

Anschließend wird eine Lösung von (1- Brom- ethyl)- benzol (0.115 ml, 0.843 mmol) in 2, 5 ml abs. MeOH, dann das Cäsiumazid (150 mg, 0.858 mmol) und weitere 2, 5 ml abs. MeOH zugegeben und über Nacht bei RT nachgerührt. Das Reaktionsgemisch wird mit MeOH verdünnt, auf Kieselgel aufgezogen und an Si- 60 flashchromatographiert.

Bedingungen Flash-Chromatographie:

Anlage: Teledyne-Isco Combi Flash RF
Säule: AnaLogix Si-60, SF25-40g
Eluent: Gradient Petrolether (PE) / Ethylacetat, Fluss: 40 ml/min, Detektion: UV 254 nm
Gradient: 0-2 min 50% PE, 2-12 min 50-100% Ethylacetat, 12-16.2 min 100% Ethylacetat

Nach dem Einengen wird das Produkt wieder in EE gelöst und zum Entfernen von TBAF- Salzen 3 x mit gesättigter $NH_4Cl$- Lösung gewaschen, über $Na_2SO_4$ getrocknet, zum Rückstand eingeengt und in Hochvakuum getrocknet. Man erhält 5- (1- Methyl- 1H- pyrazol- 4- yl)- 3- [1- (1- phenyl- ethyl)- 1H- [1, 2, 3] triazol- 4- yl]- pyrrolo [2, 3-b] pyridin- 1- carbonsäure- *tert*- butylester als einen farblosen festen Schaum (HPLC/MS: Rt=2.374 min, $[M+H]^+$ 470) .

6.2

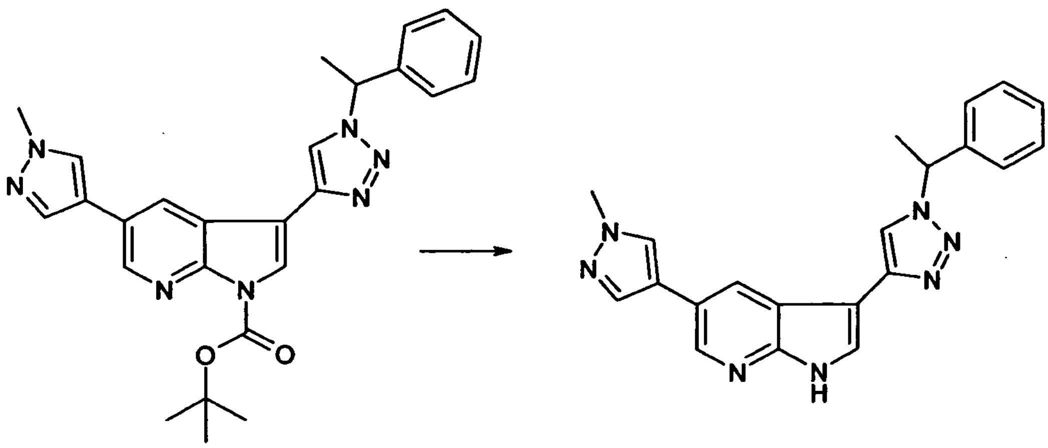

5- (1- Methyl- 1H- pyrazol- 4- yl)- 3- [1- (1- phenyl- ethyl)- 1H- [1, 2, 3] triazol- 4- yl]- pyrrolo [2, 3- b] pyridin- 1-carbonsäure- *tert*- butylester (90 mg, 0.176 mmol) wird in MeOH (5 ml) gelöst, Kaliumcarbonat (85 mg, 0.615 mmol) zugegeben und bei RT 1 h gerührt. Man versetzt mit Wasser und extrahiert dreimal mit Ethylacetat. Die organische Phase wird mit gesättigter NaCl- Lösung gewaschen, über $Na_2SO_4$ getrocknet und zum Rückstand eingeengt.

Der Rückstand wird mit Ether verrieben und abgesaugt, mit Ether gewaschen und Hochvakuum getrocknet. Man erhält 5- (1- Methyl- 1H- pyrazol- 4- yl)- 3- [1- (1- phenyl- ethyl)- 1H- [1, 2, 3] triazol- 4- yl]- 1 H- pyrrolo [2, 3- b] pyridin als farblosen Feststoff; HPLC/MS: Rt = 1.874 min, $[M+H]^+$ 370; $^1H$- NMR [DMSO- $d_6$] : $\delta$ [ppm] = 11.83 (s, 1H), 8.62 (s, 1H), 8.52 (m, 2H), 8.19 (s, 1H), 7.93 (s, 1H), 7.88 (s, 1H), 7.41- 7.32 (m, 5H), 6.01 (q, *J= 7.0 Hz*, 1H), 3.90 (s, 3H), 1.99 (d, *J= 7.03 Hz,* 3H) .

Inhibierung von PDK1

$IC_{50}$ von erfindungsgemäßen Verbindungen

| Verbindung Nr. | $IC_{50}$[PDK1] | $IC_{50}$[P-PKB T308] | $IC_{50}$ [IKKepsilon] | $IC_{50}$[TBK1] |
|---|---|---|---|---|
| "A1" | A | | | |
| "A2" | B | | | |
| "A3" | A | | | |

(fortgesetzt)

| Verbindung Nr. | $IC_{50}$[PDK1] | $IC_{50}$[P-PKB T308] | $IC_{50}$ [IKKepsilon] | $IC_{50}$[TBK1] |
|---|---|---|---|---|
| "A5" | A | B | | |
| "A6" | A | B | | |
| "A7" | A | B | A | A |
| "A8" | A | B | | |
| "A9" | A | B | A | A |
| "A15" | A | | | |
| "A16" | A | | A | A |
| "A17" | A | A | A | A |
| "A18" | A | | A | A |
| "A19" | A | B | | |
| "A20" | A | B | A | A |
| "A21" | A | B | | |
| "A22" | A | B | | |
| "A23" | A | A | | |
| "A24" | A | B | | |
| "A25" | A | A | A | A |
| "A26" | A | B | | |
| "A27" | A | | | |
| "A28" | A | | | |
| "A29" | A | B | B | A |
| "A30" | A | B | | |
| "A31" | A | B | | |
| "A32" | | B | | |
| "A33" | | | | |
| "A34" | | B | | |
| "A35" | | B | | |
| "A36" | | B | | |
| "A37" | | B | | |
| "A38" | | A | | |
| "A39" | | B | | |
| | | | | |
| $IC_{50}$: 0.5 nM - 1 $\mu$M = A 1 $\mu$M - 10 $\mu$M = B | | | | |

Die nachfolgenden Beispiele betreffen Arzneimittel:

**Beispiel A: Injektionsgläser**

Eine Lösung von 100 g eines Wirkstoffes der Formel I und 5 g Dinatriumhydrogenphosphat wird in 3 l zweifach destilliertem Wasser mit 2 N Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

**Beispiel B: Suppositorien**

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

**Beispiel C: Lösung**

Man bereitet eine Lösung aus 1 g eines Wirkstoffes der Formel I, 9,38 g $NaH_2PO_4 \cdot 2\ H_2O$, 28,48 g $Na_2HPO_4 \cdot 12\ H_2O$ und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

**Beispiel D: Salbe**

Man mischt 500 mg eines Wirkstoffes der Formel I mit 99,5 g Vaseline unter aseptischen Bedingungen.

**Beispiel E: Tabletten**

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

**Beispiel F: Dragees**

Analog Beispiel E werden Tabletten gepreßt, die anschließend in üblicher Weise mit einem Überzug aus Saccharose, Kartoffelstärke, Talk, Tragant und Farbstoff überzogen werden.

**Beispiel G: Kapseln**

2 kg Wirkstoff der Formel I werden in üblicher Weise in Hartgelatinekapseln gefüllt, so daß jede Kapsel 20 mg des Wirkstoffs enthält.

**Beispiel H: Ampullen**

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

**Patentansprüche**

**1.** Verbindungen der Formel I

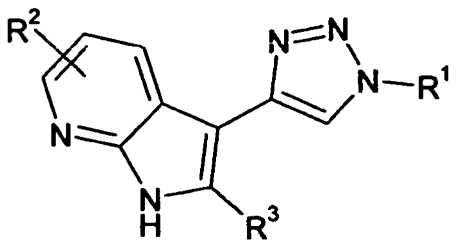

I

worin

$R^1$- C ($R^3$) ($R^4$)- Ar oder C ($R^3$) ($R^4$)- Het,
$R^2$ H, A oder- [C $(R^3)_2]_n$- Het',
$R^3$ H oder A,
$R^4$ H, -[C $(R^3)_2]_nOR^3$ oder- [C $(R^3)_2]_nCOOR^3$,
A unverzweigtes oder verzweigtes Alkyl mit 1-6 C-Atomen, worin eine $CH_2$-Gruppe durch ein O-, N oder S-Atom und/oder auch 1-7 H-Atome durch F ersetzt sein können,

Ar unsubstituiertes oder ein- oder zweifach durch Hal, $-[C(R^3)_2]_nOR^3$, $COOR^3$, CN und/ oder A substituiertes Phenyl,

Het einen unsubstituierten oder ein- oder zweifach durch A und/oder Hal substituierten ein- oder zweikernigen gesättigten aromatischen Heterocyclus mit 1 bis 4 N-, und/oder O und/oder S-Atomen,

Het' einen ein- oder zweikernigen aromatischen Heterocyclus mit 1 bis 4 N-, O- und/ oder S- Atomen, der unsubstituiert ist oder ein- oder zweifach durch A und/ oder $[C(R^3)_2]_nHet^1$ substituiert sein kann,

$Het^1$ einen einkernigen gesättigten Heterocyclus mit 1 bis 2 N und/oder O-Atomen, der ein- oder zweifach durch A sein kann,

Hal F, Cl, Br oder I

n 0, 1 oder 2

bedeuten,

sowie ihre pharmazeutisch verwendbaren Salze, Tautomere und Stereoisomere, einschließlich deren Mischungen in allen Verhältnissen.

**2.** Verbindungen nach Anspruch 1, ausgewählt aus der Gruppe

| Verbindung Nr. | Name und/oder Struktur |
|---|---|
| "A1" | 3-(1-Benzyl-1H-[1,2,3]triazol-4-yl)-1H-pyrrolo[2,3-b]pyridin |
| "A2" | 3-[1-(4-Chlorbenzyl)-1H-[1,2,3]triazol-4-yl]-1H-pyrrolo[2,3-b]pyridin |
| "A3" | 3-(1-Benzyl-1H-[1,2,3]triazol-4-yl)-2-methyl-1H-pyrrolo[2,3-b]pyridin |
| "A4" | 3-(1-Benzyl-1H-[1,2,3]triazol-4-yl)-5-(1-methyl-1H-pyrazol-4-yl)-1H-pyrrolo[2,3-b]pyridin |
| "A5" | 3-[1-(3-Fluor-benzyl)-1H-[1,2,3]triazol-4-yl]-5-(1-methyl-1H-pyrazol-4-yl)-1H-pyrrolo[2,3-b]pyridin |
| "A6" | 3-[1-(4-Fluor-benzyl)-1H-[1,2,3]triazol-4-yl]-5-(1-methyl-1H-pyrazol-4-yl)-1H-pyrrolo[2,3-b]pyridin |
| "A7" | 3-[1-(3-Methyl-benzyl)-1H-[1,2,3]triazol-4-yl]-5-(1-methyl-1H-pyrazol-4-yl)-1H-pyrrolo[2,3-b]pyridin |
| "A8" | 5-(1-Methyl-1H-pyrazol-4-yl)-3-[1-(1-phenyl-ethyl)-1H-[1,2,3]triazol-4-yl]-1H-pyrrolo[2,3-b]pyridin |
| "A9" | 5-(1-Methyl-1H-pyrazol-4-yl)-3-(1-pyridin-4-ylmethyl-1H-[1,2,3]triazol-4-yl)-1H-pyrrolo[2,3-b]pyridin |
| "A10" | 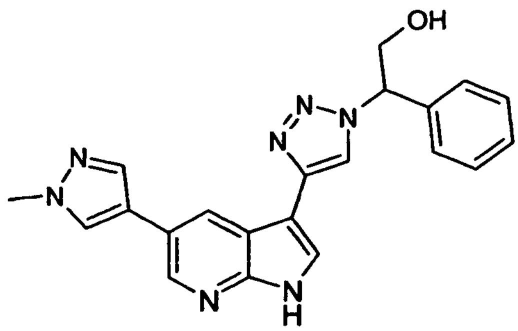 |
| "A11" | 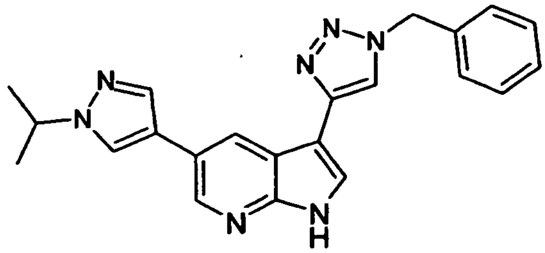 |

(fortgesetzt)

| Verbindung Nr. | Name und/oder Struktur |
|---|---|
| "A12" | 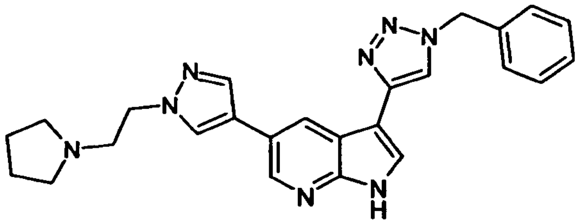 |
| "A13" | 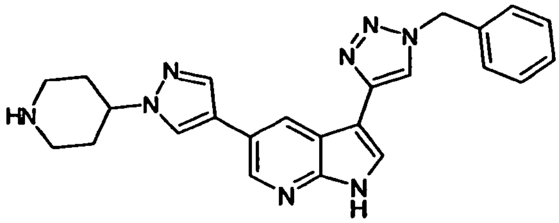 |
| "A14" | 2-{4-[3-(1-Benzyl-1H-[1,2,3]triazol-4-yl)-1H-pyrrolo[2,3-b]pyridin-5-yl]-pyrazol-1-yl}-ethanol |
| "A15" | 3-(1-Benzyl-1H-[1,2,3]triazol-4-yl)-4-methoxy-1H-pyrrolo[2,3-b]pyridin |
| "A16" | 5-(1-Methyl-1H-pyrazol-4-yl)-3-(1-phenethyl-1H-[1,2,3]triazol-4-yl)-1H-pyrrolo[2,3-b]pyridin |
| "A17" | 3-[1-(2-Fluor-benzyl)-1H-[1,2,3]triazol-4-yl]-5-(1-methyl-1H-pyrazol-4-yl)-1H-pyrrolo[2,3-b]pyridin |
| "A18" | 5-(1-Methyl-1H-pyrazol-4-yl)-3-[1-(2-morpholin-4-yl-ethyl)-1H-[1,2,3]triazol-4-yl]-1H-pyrrolo[2,3-b]pyridin |
| "A19" | 5-(1-Methyl-1H-pyrazol-4-yl)-3-{1-[1-(3-trifluormethyl-phenyl)-ethyl]-1H-[1,2,3]triazol-4-yl}-1H-pyrrolo[2,3-b]pyridin |
| "A20" | 5-(1-Methyl-1H-pyrazol-4-yl)-3-{1-[1-(2-trifluormethyl-phenyl)-ethyl]-1H-[1,2,3]triazol-4-yl}-1H-pyrrolo[2,3-b]pyridin |
| "A21" | 3-[1-(2,4-Difluor-benzyl)-1H-[1,2,3]triazol-4-yl]-5-(1-methyl-1 H-pyrazol-4-yl)-1 H-pyrrolo[2,3-b] pyridin |
| "A22" | 3-[1-(2-Fluor-3-trifluormethyl-benzyl)-1H-[1,2,3]triazol-4-yl]-5-(1-methyl-1H-pyrazol-4-yl)-1H-pyrrolo[2,3-b]pyridin |
| "A23" | 3-[1-(3-Chlor-2-fluor-benzyl)-1H-[1,2,3]triazol-4-yl]-5-(1-methyl-1H-pyrazol-4-yl)-1H-pyrrolo[2,3-b]pyridin |
| "A24" | 3-[1-(2-Fluor-6-trifluormethyl-benzyl)-1H-[1,2,3]triazol-4-yl]-5-(1-methyl-1H-pyrazol-4-yl)-1H-pyrrolo[2,3-b]pyridin |
| "A25" | 3-[1-(2,3-Difluor-benzyl)-1H-[1,2,3]triazol-4-yl]-5-(1-methyl-1H-pyrazol-4-yl)-1H-pyrrolo[2,3-b] pyridin |
| "A26" | 3-[1-(2-Fluor-4-trifluormethyl-benzyl)-1H-[1,2,3]triazol-4-yl]-5-(1-methyl-1H-pyrazol-4-yl)-1H-pyrrolo[2,3-b]pyridin |
| "A27" | 4-(1-{4-[5-(1-Methyl-1H-pyrazol-4-yl)-1H-pyrrolo[2,3-b]pyridin-3-yl]-[1,2,3]triazol-1-yl}-ethyl)-benzoesäure |
| "A28" | 3-{4-[5-(1-Methyl-1H-pyrazol-4-yl)-1H-pyrrolo[2,3-b]pyridin-3-yl]-[1,2,3]triazol-1-yl}-3-phenyl-propionsäure |
| "A29" | 6-{4-[5-(1-Methyl-1H-pyrazol-4-yl)-1H-pyrrolo[2,3-b]pyridin-3-yl]-[1,2,3]triazol-1-ylmethyl}-chinolin |
| "A30" | [4-(1-{4-[5-(1-Methyl-1H-pyrazol-4-yl)-1H-pyrrolo[2,3-b]pyridin-3-yl]-[1,2,3]triazol-1-yl}-ethyl)-phenyl]-methanol |

(fortgesetzt)

| Verbindung Nr. | Name und/oder Struktur |
|---|---|
| "A31" | 2-{4-[5-(1-Methyl-1H-pyrazol-4-yl)-1H-pyrrolo[2,3-b]pyridin-3-yl]-[1,2,3]triazol-1-yl}-1-phenyl-ethanol |
| "A32" | 3-[1-(3-Fluor-pyridin-4-ylmethyl)-1H-[1,2,3]triazol-4-yl]-5-(1-methyl-1H-pyrazol-4-yl)-1H-pyrrolo [2,3-b]pyridin |
| "A33" | 3-[1-(2-Chlor-benzyl)-1H-[1,2,3]triazol-4-yl]-5-(1-methyl-1H-pyrazol-4-yl)-1H-pyrrolo[2,3-b]pyridin |
| "A34" | 3-[1-(3-Methoxy-benzyl)-1H-[1,2,3]triazol-4-yl]-5-(1-methyl-1H-pyrazol-4-yl)-1H-pyrrolo[2,3-b] pyridin |
| "A35" | (2-{4-[5-(1-Methyl-1H-pyrazol-4-yl)-1H-pyrrolo[2,3-b]pyridin-3-yl]-[1,2,3]triazol-1-ylmethyl}-phenyl)-methanol |
| "A36" | 3-[1-(4-Difluormethoxy-benzyl)-1H-[1,2,3]triazol-4-yl]-5-(1-methyl-1H-pyrazol-4-yl)-1H-pyrrolo [2,3-b]pyridin |
| "A37" | 3-{4-[5-(1-Methyl-1H-pyrazol-4-yl)-1H-pyrrolo[2,3-b]pyridin-3-yl]-[1,2,3]triazol-1-ylmethyl}-benzoesäure-methylester |
| "A38" | 2-{4-[5-(1-Methyl-1H-pyrazol-4-yl)-1H-pyrrolo[2,3-b]pyridin-3-yl]-[1,2,3]triazol-1-ylmethyl}-benzonitril |
| "A39" | 3-[1-(3-Difluormethoxy-benzyl)-1H-[1,2,3]triazol- 4-yl]-5-(1-methyl-1H-pyrazol-4-yl)-1H-pyrrolo [2,3-b]pyridin |
| "A40" | 3-{4-[5-(1-Methyl-1H-pyrazol-4-yl)-1H-pyrrolo[2,3- b]pyridin-3-yl]-[1,2,3-triazol-1-yl}-3-phenyl-propan-1-ol |

sowie ihre pharmazeutisch verwendbaren Salze, Tautomere und Stereoisomere, einschließlich deren Mischungen in allen Verhältnissen.

**3.** Verfahren zur Herstellung von Verbindungen der Formel I nach den Ansprüchen 1-2 sowie ihrer pharmazeutisch verwendbaren Salze, Tautomeren und Stereoisomeren, **dadurch gekennzeichnet, daß** man
aus einer Verbindung der Formel II

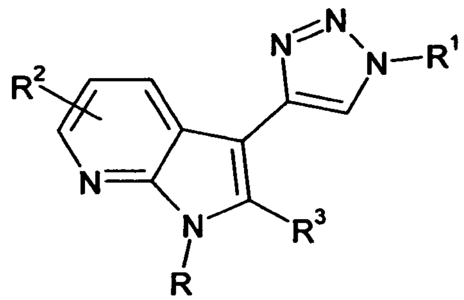

II

,

worin R eine Indolschutzgruppe bedeutet,
die Indolschutzgruppe abspaltet,
und/oder
eine Base oder Säure der Formel I in eines ihrer Salze umwandelt.

**4.** Arzneimittel, enthaltend mindestens eine Verbindung der Formel I gemäß Anspruch 1-2 und/oder ihre pharmazeutisch verwendbaren Salze, Tautomeren und Stereoisomeren, einschließlich deren Mischungen in allen Verhältnissen, sowie gegebenenfalls Träger- und/oder Hilfsstoffe.

**5.** Verbindungen der Formel I gemäß Anspruch 1-2 sowie ihrer pharmazeutisch verwendbaren Salze, Tautomeren und Stereoisomeren, einschließlich deren Mischungen in allen Verhältnissen, zur Verwendung zur Behandlung von Tumoren, Tumorwachstum, Tumormetastasen und/oder AIDS.

6. Verwendung von Verbindungen der Formel I gemäß Anspruch 1-2 sowie ihrer pharmazeutisch verwendbaren Salze, Tautomeren und Stereoisomeren, einschließlich deren Mischungen in allen Verhältnissen, zur Herstellung eines Arzneimittels zur Behandlung von Tumoren, Tumorwachstum, Tumormetastasen und/oder AIDS.

7. Verwendung von Verbindungen der Formel I gemäß Anspruch 1- 2 und/ oder ihrer physiologisch unbedenklichen Salze, Tautomeren und Stereoisomeren zur Herstellung eines Arzneimittels zur Behandlung von Tumoren, wobei eine therapeutisch wirksame Menge einer Verbindung der Formel I in Kombination mit einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl- Proteintransferasehemmer, 7) HMG- CoA- Reduktase- Hemmer, 8) HIV-Protease- Hemmer, 9) Reverse- Transkriptase- Hemmer sowie 10) weiterer Angiogenese- Hemmer verabreicht wird.

8. Verwendung von Verbindungen der Formel I gemäß Anspruch 1- 2 und/ oder ihrer physiologisch unbedenklichen Salze, Tautomeren und Stereoisomeren zur Herstellung eines Arzneimittels zur Behandlung von Tumoren wobei eine therapeutisch wirksame Menge einer Verbindung der Formel I ihn Kombination mit Radiotherapie und einer Verbindung aus der Gruppe 1) Östrogenrezeptormodulator, 2) Androgenrezeptor- modulator, 3) Retinoidrezeptormodulator, 4) Zytotoxikum, 5) antiproliferatives Mittel, 6) Prenyl- Proteintransferasehemmer, 7) HMG- CoA- Reduktase- Hemmer, 8) HIV- Protease- Hemmer, 9) Reverse- Transkriptase- Hemmer sowie 10) weiterer Angiogenese-Hemmer verabreicht wird.

**Claims**

1. Compounds of the formula I

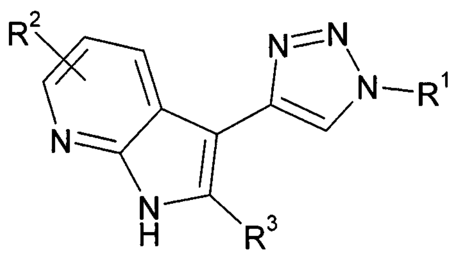

I

in which

$R^1$ denotes- C ($R^3$) ($R^4$)- Ar or C ($R^3$) ($R^4$)- Het,
$R^2$ denotes H, A or- [C $(R^3)_2]_n$- Het',
$R^3$ denotes H or A,
$R^4$ denotes H, -[C $(R^3)_2]_n$O$R^3$ or- [C $(R^3)_2]_n$COO$R^3$,
A denotes unbranched or branched alkyl having 1-6 C atoms, in which one $CH_2$ group may be replaced by an O, N or S atom and/or, in addition, 1-7 H atoms may be replaced by F,
Ar denotes phenyl which is unsubstituted or mono- or disubstituted by Hal, -[C ($R^3$) $]_n$O$R^3$, COO$R^3$, CN and/or A,
Het denotes a mono- or bicyclic saturated aromatic heterocycle having 1 to 4 N and/or O and/or S atoms which is unsubstituted or mono- or disubstituted by A and/or Hal,
Het' denotes a mono- or bicyclic aromatic heterocycle having 1 to 4 N, O and/or S atoms which is unsubstituted or may be mono- or disubstituted by A and/or [C $(R^3)_2]_n$$Het^1$,
$Het^1$ denotes a monocyclic saturated heterocycle having 1 to 2 N and/or O atoms, which may be mono- or disubstituted by A,
Hal denotes F, Cl, Br or I,
n denotes 0, 1 or 2,

and pharmaceutically usable salts, tautomers and stereoisomers thereof, including mixtures thereof in all ratios.

2. Compounds according to Claim 1, selected from the group

| Compound No. | Name and/or structure |
|---|---|
| "A1" | 3-(1-Benzyl-1H-1,2,3-triazol-4-yl)-1H-pyrrolo-[2,3-b]pyridine |

(continued)

| Compound No. | Name and/or structure |
|---|---|
| "A2" | 3-[1-(4-Chlorobenzyl)-1H-1,2,3-triazol-4-yl]-1H-pyrrolo[2,3-b]pyridine |
| "A3" | 3-(1-Benzyl-1H-1,2,3-triazol-4-yl)-2-methyl-1H-pyrrolo[2,3-b]pyridine |
| "A4" | 3-(1-Benzyl-1H-1,2,3-triazol-4-yl)-5-(1-methyl-1H-pyrazol-4-yl)-1H-pyrrolo[2,3-b]pyridine |
| "A5" | 3-[1-(3-Fluorobenzyl)-1H-1,2,3-triazol-4-yl]-5-(1-methyl-1H-pyrazol-4-yl)-1H-pyrrolo[2,3-b] pyridine |
| "A6" | 3-[1-(4-Fluorobenzyl)-1H-1,2,3-triazol-4-yl]-5-(1-methyl-1H-pyrazol-4-yl)-1H-pyrrolo[2,3-b] pyridine |
| "A7" | 3-[1-(3-Methylbenzyl)-1H-1,2,3-triazol-4-yl]-5-(1-methyl-1H-pyrazol-4-yl)-1H-pyrrolo[2,3-b] pyridine |
| "A8" | 5-(1-Methyl-1H-pyrazol-4-yl)-3-[1-(1-phenylethyl)-1H-1,2,3-triazol-4-yl]-1H-pyrrolo[2,3-b]pyridine |
| "A9" | 5-(1-Methyl-1H-pyrazol-4-yl)-3-(1-pyridin-4-yl-methyl-1H-1,2,3-triazol-4-yl)-1H-pyrrolo[2,3-b]-pyridine |
| "A10" | 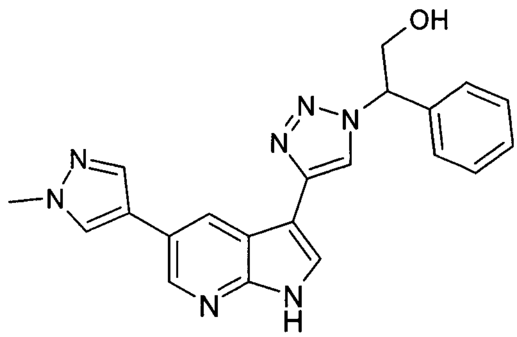 |
| "A11" | 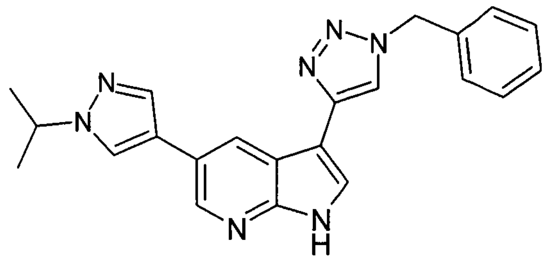 |
| "A12" | 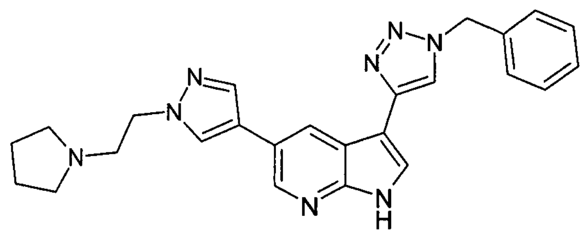 |
| "A13" | 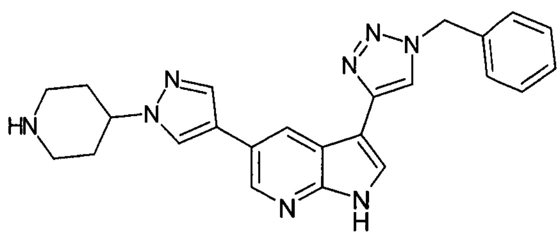 |
| "A14" | 2-{4-[3-(1-Benzyl-1H-1,2,3-triazol-4-yl)-1H-pyrrolo-[2,3-b]pyridin-5-yl]pyrazol-1-yl}ethanol |
| "A15" | 3-(1-Benzyl-1H-1,2,3-triazol-4-yl)-4-methoxy-1H-pyrrolo[2,3-b]pyridine |

(continued)

| Compound No. | Name and/or structure |
|---|---|
| "A16" | 5-(1-Methyl-1H-pyrazol-4-yl)-3-(1-phenethyl-1H-1,2,3-triazol-4-yl)-1H-pyrrolo[2,3-b]pyridine |
| "A17" | 3-[1-(2-Fluorobenzyl)-1H-1,2,3-triazol-4-yl]-5-(1-methyl-1H-pyrazol-4-yl)-1H-pyrrolo[2,3-b] pyridine |
| "A18" | 5-(1-Methyl-1H-pyrazol-4-yl)-3-[1-(2-morpholin-4-ylethyl)-1H-1,2,3-triazol-4-yl]-1H-pyrrolo[2,3-b]-pyridine |
| "A19" | 5-(1-Methyl-1H-pyrazol-4-yl)-3-{1-[1-(3-trifluoromethylphenyl)ethyl]-1H-1,2,3-triazol-4-yl}-1H-pyrrolo[2,3-b]pyridine |
| "A20" | 5-(1-Methyl-1H-pyrazol-4-yl)-3-{1-[1-(2-trifluoromethylphenyl)ethyl]-1H-1,2,3-triazol-4-yl}-1H-pyrrolo[2,3-b]pyridine |
| "A21" | 3-[1-(2,4-Difluorobenzyl)-1H-1,2,3-triazol-4-yl]-5-(1-methyl-1H-pyrazol-4-yl)-1H-pyrrolo[2,3-b]-pyridine |
| "A22" | 3-[1-(2-Fluoro-3-trifluoromethylbenzyl)-1H-1,2,3-triazol-4-yl]-5-(1-methyl-1H-pyrazol-4-yl)-1H-pyrrolo[2,3-b]pyridine |
| "A23" | 3-[1-(3-Chloro-2-fluorobenzyl)-1H-1,2,3-triazol-4-yl]-5-(1-methyl-1H-pyrazol-4-yl)-1H-pyrrolo [2,3-b]-pyridine |
| "A24" | 3-[1-(2-Fluoro-6-trifluoromethylbenzyl)-1H-1,2,3-triazol-4-yl]-5-(1-methyl-1H-pyrazol-4-yl)-1H-pyrrolo[2,3-b]pyridine |
| "A25" | 3-[1-(2,3-Difluorobenzyl)-1H-1,2,3-triazol-4-yl]-5-(1-methyl-1H-pyrazol-4-yl)-1H-pyrrolo[2,3-b]-pyridine |
| "A26" | 3-[1-(2-Fluoro-4-trifluoromethylbenzyl)-1H-1,2,3-triazol-4-yl]-5-(1-methyl-1H-pyrazol-4-yl)-1H-pyrrolo[2,3-b]pyridine |
| "A27" | 4-(1-{4-[5-(1-Methyl-1H-pyrazol-4-yl)-1H-pyrrolo-[2,3-b]pyridin-3-yl]-1,2,3-triazol-1-yl}ethyl) benzoic acid |
| "A28" | 3-{4-[5-(1-Methyl-1H-pyrazol-4-yl)-1H-pyrrolo-[2,3-b]pyridin-3-yl]-1,2,3-triazol-1-yl}-3-phenylpropionic acid |
| "A29" | 6-{4-[5-(1-Methyl-1H-pyrazol-4-yl)-1H-pyrrolo-[2,3-b]pyridin-3-yl]-1,2,3-triazol-1-ylmethyl}-quinoline |
| "A30" | [4-(1-{4-[5-(1-Methyl-1H-pyrazol-4-yl)-1H-pyrrolo-[2,3-b]pyridin-3-yl]-1,2,3-triazol-1-yl}ethyl) phenyl]-methanol |
| "A31" | 2-{4-[5-(1-Methyl-1H-pyrazol-4-yl)-1H-pyrrolo-[2,3-b]pyridin-3-yl]-1,2,3-triazol-1-yl}-1-phenylethanol |
| "A32" | 3-[1-(3-Fluoropyridin-4-ylmethyl)-1H-1,2,3-triazol-4-yl]-5-(1-methyl-1H-pyrazol-4-yl)-1H-pyrrolo-[2,3-b]pyridine |
| "A33" | 3-[1-(2-Chlorobenzyl)-1H-1,2,3-triazol-4-yl]-5-(1-methyl-1H-pyrazol-4-yl)-1H-pyrrolo[2,3-b] pyridine |
| "A34" | 3-[1-(3-Methoxybenzyl)-1H-1,2,3-triazol-4-yl]-5-(1-methyl-1H-pyrazol-4-yl)-1H-pyrrolo[2,3-b] pyridine |
| "A35" | (2-{4-[5-(1-Methyl-1H-pyrazol-4-yl)-1H-pyrrolo-[2,3-b]pyridin-3-yl]-1,2,3-triazol-1-ylmethyl} phenyl)-methanol |
| "A36" | 3-[1-(4-Difluoromethoxybenzyl)-1H-1,2,3-triazol-4-yl]-5-(1-methyl-1H-pyrazol-4-yl)-1H-pyrrolo [2,3-b]-pyridine |
| "A37" | Methyl 3-{4-[5-(1-methyl-1H-pyrazol-4-yl)-1H-pyrrolo[2,3-b]pyridin-3-yl]-1,2,3-triazol-1-ylmethyl}-benzoate |

(continued)

| Compound No. | Name and/or structure |
|---|---|
| "A38" | 2-{4-[5-(1-Methyl-1H-pyrazol-4-yl)-1H-pyrrolo-[2,3-b]pyridin-3-yl]-1,2,3-triazol-1-ylmethyl}-benzonitrile |
| "A39" | 3-[1-(3-Difluoromethoxybenzyl)-1H-1,2,3-triazol-4-yl]-5-(1-methyl-1H-pyrazol-4-yl)-1H-pyrrolo [2,3-b]-pyridine |
| "A40" | 3-{4-[5-(1-Methyl-1H-pyrazol-4-yl)-1H-pyrrolo-[2,3-b]pyridin-3-yl]-1,2,3-triazol-1-yl}-3-phenyl-propan-1-ol |

and pharmaceutically usable salts, tautomers and stereoisomers thereof, including mixtures thereof in all ratios.

3. Process for the preparation of compounds of the formula I according to Claims 1-2 and pharmaceutically usable salts, tautomers and stereoisomers thereof, **characterised in that**
the indole-protecting group is cleaved off from a compound of the formula II

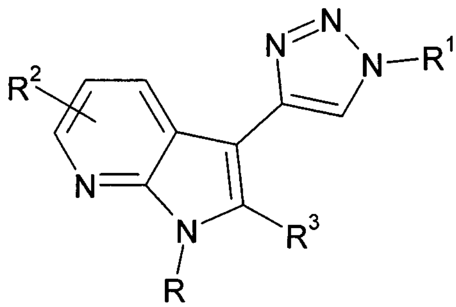

II

,

in which R denotes an indole-protecting group,
and/or
a base or acid of the formula I is converted into one of its salts.

4. Medicaments comprising at least one compound of the formula I according to Claims 1-2 and/or pharmaceutically usable salts, tautomers and stereoisomers thereof, including mixtures thereof in all ratios, and optionally excipients and/or adjuvants.

5. Compounds of the formula I according to Claims 1-2 and pharmaceutically usable salts, tautomers and stereoisomers thereof, including mixtures thereof in all ratios, for use for the treatment of tumours, tumour growth, tumour metastases and/or AIDS.

6. Use of compounds of the formula I according to Claims 1-2 and pharmaceutically usable salts, tautomers and stereoisomers thereof, including mixtures thereof in all ratios, for the preparation of a medicament for the treatment of tumours, tumour growth, tumour metastases and/or AIDS.

7. Use of compounds of the formula I according to Claims 1-2 and/or physiologically acceptable salts, tautomers and stereoisomers thereof for the preparation of a medicament for the treatment of tumours, where a therapeutically effective amount of a compound of the formula I is administered in combination with a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

8. Use of compounds of the formula I according to Claims 1-2 and/or physiologically acceptable salts, tautomers and stereoisomers thereof for the preparation of a medicament for the treatment of tumours, where a therapeutically effective amount of a compound of the formula I is administered in combination with radiotherapy and a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

## Revendications

1. Composés de formule I

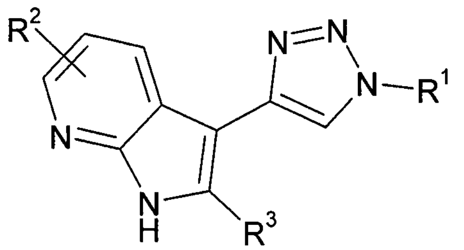

I

dans laquelle

$R^1$ désigne- C ($R^3$) ($R^4$)- Ar ou C ($R^3$) ($R^4$)- Hét,
$R^2$ désigne H, A ou- [C $(R^3)_2]_n$- Hét',
$R^3$ désigne H ou A,
$R^4$ désigne H, -[C $(R^3)_2]_n OR^3$ ou- [C $(R^3)_2]_n COOR^3$,
A désigne alkyle non ramifié ou ramifié ayant 1-6 atomes de C, où un groupement $CH_2$ peut être remplacé par un atome de O, N ou S et/ou, de plus, 1-7 atomes de H peuvent être remplacés par F,
Ar désigne phényle qui est non substitué ou mono- ou disubstitué par Hal, -[C $(R^3)_2]_n OR^3$, $COOR^3$, CN et/ou A,
Hét désigne un hétérocycle aromatique saturé mono- ou bicyclique ayant 1 à 4 atomes de N et/ou O et/ou S qui est non substitué ou mono- ou disubstitué par A et/ou Hal,
Hét' désigne un hétérocycle aromatique mono- ou bicyclique ayant 1 à 4 atomes de N, O et/ou S qui est non substitué ou peut être mono- ou disubstitué par A et/ou [C $(R^3)_2]_n$Hét$^1$,
Hét$^1$ désigne un hétérocycle saturé monocyclique ayant 1 à 2 atomes de N et/ou O, qui peut être mono- ou disubstitué par A,
Hal désigne F, Cl, Br ou I,
n désigne 0, 1 ou 2,

et les sels, tautomères et stéréoisomères pharmaceutiquement utilisables de ceux-ci, y compris des mélanges de ceux-ci en toutes proportions.

2. Composés selon la revendication 1, choisis dans le groupe constitué par

| Composé n° | Nom et/ou structure |
|---|---|
| "A1" | 3-(1-Benzyl-1H-1,2,3-triazol-4-yl)-1H-pyrrolo-[2,3-b]pyridine |
| "A2" | 3-[1-(4-Chlorobenzyl)-1H-1,2,3-triazol-4-yl]-1H-pyrrolo[2,3-b]pyridine |
| "A3" | 3-(1-Benzyl-1H-1,2,3-triazol-4-yl)-2-méthyl-1H-pyrrolo[2,3-b]pyridine |
| "A4" | 3-(1-Benzyl-1H-1,2,3-triazol-4-yl)-5-(1-méthyl-1H-pyrazol-4-yl)-1H-pyrrolo[2,3-b]pyridine |
| "A5" | 3-[1-(3-Fluorobenzyl)-1H-1,2,3-triazol-4-yl]-5-(1-méthyl-1H-pyrazol-4-yl)-1H-pyrrolo[2,3-b]pyridine |
| "A6" | 3-[1-(4-Fluorobenzyl)-1H-1,2,3-triazol-4-yl]-5-(1-méthyl-1H-pyrazol-4-yl)-1H-pyrrolo[2,3-b]pyridine |
| "A7" | 3-[1-(3-Méthylbenzyl)-1H-1,2,3-triazol-4-yl]-5-(1-méthyl-1H-pyrazol-4-yl)-1H-pyrrolo[2,3-b]pyridine |
| "A8" | 5-(1-Méthyl-1H-pyrazol-4-yl)-3-[1-(1-phényléthyl)-1H-1,2,3-triazol-4-yl]-1H-pyrrolo[2,3-b]pyridine |
| "A9" | 5-(1-Méthyl-1H-pyrazol-4-yl)-3-(1-pyridin-4-yl-méthyl-1H-1,2,3-triazol-4-yl)-1H-pyrrolo[2,3-b]-pyridine |

(suite)

| Composé n° | Nom et/ou structure |
|---|---|
| "A10" | 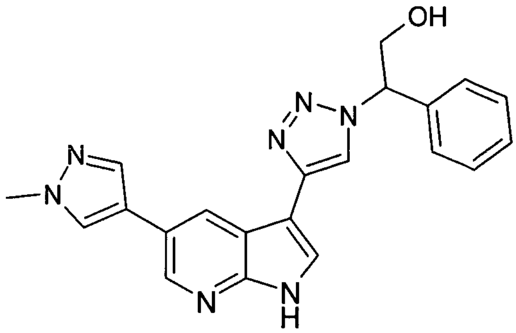 |
| "A11" | 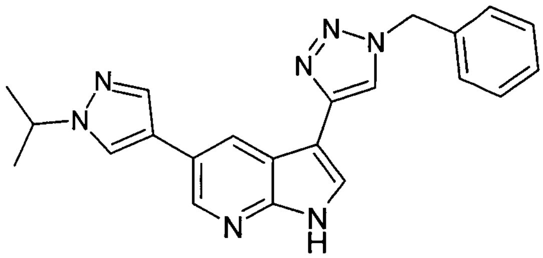 |
| "A12" | 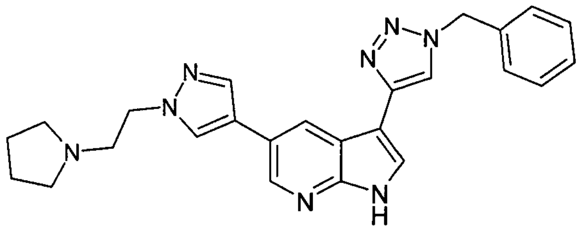 |
| "A13" | 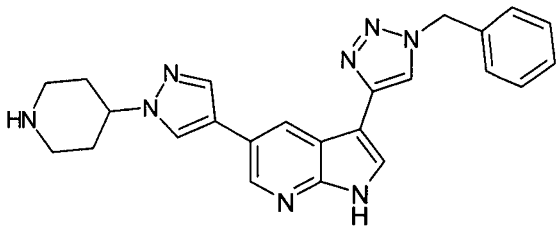 |
| "A14" | 2-{4-[3-(1-Benzyl-1H-1,2,3-triazol-4-yl)-1H-pyrrolo[2,3-b]pyridin-5-yl]pyrazol-1-yl}éthanol |
| "A15" | 3-(1-Benzyl-1H-1,2,3-triazol-4-yl)-4-méthoxy-1H-pyrrolo[2,3-b]pyridine |
| "A16" | 5-(1-Méthyl-1H-pyrazol-4-yl)-3-(1-phénéthyl-1H-1,2,3-triazol-4-yl)-1H-pyrrolo[2,3-b]pyridine |
| "A17" | 3-[1-(2-Fluorobenzyl)-1H-1,2,3-triazol-4-yl]-5-(1-méthyl-1H-pyrazol-4-yl)-1H-pyrrolo[2,3-b]pyridine |
| "A18" | 5-(1-Méthyl-1H-pyrazol-4-yl)-3-[1-(2-morpholin-4-yléthyl)-1H-1,2,3-triazol-4-yl]-1H-pyrrolo[2,3-b]-pyridine |
| "A19" | 5-(1-Méthyl-1H-pyrazol-4-yl)-3-{1-[1-(3-trifluorométhylphényl)éthyl]-1H-1,2,3-triazol-4-yl}-1H-pyrrolo [2,3-b]pyridine |
| "A20" | 5-(1-Méthyl-1H-pyrazol-4-yl)-3-{1-[1-(2-trifluorométhylphényl)éthyl]-1H-1,2,3-triazol-4-yl}-1H-pyrrolo [2,3-b]pyridine |
| "A21" | 3-[1-(2,4-Difluorobenzyl)-1H-1,2,3-triazol-4-yl]-5-(1-méthyl-1H-pyrazol-4-yl)-1H-pyrrolo[2,3-b] pyridine |
| "A22" | 3-[1-(2-Fluoro-3-trifluorométhylbenzyl)-1H-1,2,3-triazol-4-yl]-5-(1-méthyl-1H-pyrazol-4-yl)-1H-pyrrolo[2,3-b]pyridine |

(suite)

| Composé n° | Nom et/ou structure |
|---|---|
| "A23" | 3-[1-(3-Chloro-2-fluorobenzyl)-1H-1,2,3-triazol-4-yl]-5-(1-méthyl-1H-pyrazol-4-yl)-1H-pyrrolo[2,3-b]pyridine |
| "A24" | 3-[1-(2-Fluoro-6-trifluorométhylbenzyl)-1H-1,2,3-triazol-4-yl]-5-(1-méthyl-1H-pyrazol-4-yl)-1H-pyrrolo[2,3-b]pyridine |
| "A25" | 3-[1-(2,3-Difluorobenzyl)-1H-1,2,3-triazol-4-yl]-5-(1-méthyl-1H-pyrazol-4-yl)-1H-pyrrolo[2,3-b]pyridine |
| "A26" | 3-[1-(2-Fluoro-4-trifluorométhylbenzyl)-1H-1,2,3-triazol-4-yl]-5-(1-méthyl-1H-pyrazol-4-yl)-1H-pyrrolo[2,3-b]pyridine |
| "A27" | Acide 4-(1-{4-[5-(1-méthyl-1H-pyrazol-4-yl)-1H-pyrrolo[2,3-b]pyridin-3-yl]-1,2,3-triazol-1-yl}-éthyl)benzoïque |
| "A28" | Acide 3-{4-[5-(1-méthyl-1H-pyrazol-4-yl)-1H-pyrrolo[2,3-b]pyridin-3-yl]-1,2,3-triazol-1-yl}-3-phénylpropionique |
| "A29" | 6-{4-[5-(1-Méthyl-1H-pyrazol-4-yl)-1H-pyrrolo[2,3-b]pyridin-3-yl]-1,2,3-triazol-1-yl-méthyl}quinoléine |
| "A30" | [4-(1-{4-[5-(1-Méthyl-1H-pyrazol-4-yl)-1H-pyrrolo[2,3-b]pyridin-3-yl]-1,2,3-triazol-1-yl}-éthyl)phényl]méthanol |
| "A31" | 2-{4-[5-(1-Méthyl-1H-pyrazol-4-yl)-1 H-pyrrolo[2,3-b]pyridin-3-yl]-1,2,3-triazol-1-yl}-1-phényléthanol |
| "A32" | 3-[1-(3-Fluoropyridin-4-ylméthyl)-1H-1,2,3-triazol-4-yl]-5-(1-méthyl-1H-pyrazol-4-yl)-1H-pyrrolo[2,3-b]pyridine |
| "A33" | 3-[1-(2-Chlorobenzyl)-1H-1,2,3-triazol-4-yl]-5-(1-méthyl-1H-pyrazol-4-yl)-1H-pyrrolo[2,3-b]pyridine |
| "A34" | 3-[1-(3-Méthoxybenzyl)-1H-1,2,3-triazol-4-yl]-5-(1-méthyl-1H-pyrazol-4-yl)-1H-pyrrolo[2,3-b]pyridine |
| "A35" | (2-{4-[5-(1-Méthyl-1H-pyrazol-4-yl)-1H-pyrrolo[2,3-b]pyridin-3-yl]-1,2,3-triazol-1-yl-méthyl}phényl)méthanol |
| "A36" | 3-[1-(4-Difluorométhoxybenzyl)-1H-1,2,3-triazol-4-yl]-5-(1-méthyl-1H-pyrazol-4-yl)-1H-pyrrolo[2,3-b]pyridine |
| "A37" | 3-{4-[5-(1-Méthyl-1H-pyrazol-4-yl)-1H-pyrrolo[2,3-b]pyridin-3-yl]-1,2,3-triazol-1-yl-méthyl}benzoate de méthyle |
| "A38" | 2-{4-[5-(1-Méthyl-1H-pyrazol-4-yl)-1H-pyrrolo[2,3-b]pyridin-3-yl]-1,2,3-triazol-1-yl-méthyl}benzonitrile |
| "A39" | 3-[1-(3-Difluorométhoxybenzyl)-1H-1,2,3-triazol-4-yl]-5-(1-méthyl-1H-pyrazol-4-yl)-1H-pyrrolo[2,3-b]pyridine |
| "A40" | 3-{4-[5-(1-Méthyl-1H-pyrazol-4-yl)-1H-pyrrolo-[2,3-b]pyridin-3-yl]-1,2,3-triazol-1-yl}-3-phényl-propan-1-ol |

et les sels, tautomères et stéréoisomères pharmaceutiquement utilisables de ceux-ci, y compris des mélanges de ceux-ci en toutes proportions.

**3.** Procédé de préparation de composés de formule I selon les revendications 1-2 et de sels, tautomères et stéréoisomères pharmaceutiquement utilisables de ceux-ci, **caractérisé en ce que**
le groupement de protection d'indole est éliminé par clivage d'un composé de formule II

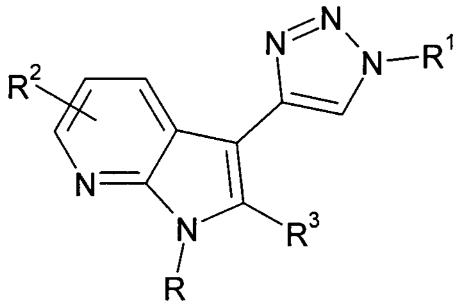

II

,

dans laquelle R désigne un groupement de protection d'indole,
et/ou
une base ou un acide de formule I est converti(e) en l'un de ses sels.

**4.** Médicaments comprenant au moins un composé de formule I selon les revendications 1-2 et/ou des sels, tautomères et stéréoisomères pharmaceutiquement utilisables de celui-ci, y compris des mélanges de ceux-ci en toutes proportions, et éventuellement des excipients et/ou des adjuvants.

**5.** Composés de formule I selon les revendications 1-2 et des sels, tautomères et stéréoisomères pharmaceutiquement utilisables de ceux-ci, y compris des mélanges de ceux-ci en toutes proportions, pour une utilisation pour le traitement de tumeurs, de la croissance tumorale, de métastases tumorales et/ou du SIDA.

**6.** Utilisation de composés de formule I selon les revendications 1-2 et de sels, tautomères et stéréoisomères pharmaceutiquement utilisables de ceux-ci, y compris des mélanges de ceux-ci en toutes proportions, pour la préparation d'un médicament destiné au traitement de tumeurs, de la croissance tumorale, de métastases tumorales et/ou du SIDA.

**7.** Utilisation de composés de formule I selon les revendications 1-2, et/ou de sels, tautomères et stéréoisomères physiologiquement acceptables de ceux-ci, pour la préparation d'un médicament destiné au traitement de tumeurs, dans laquelle une quantité thérapeutiquement efficace d'un composé de formule I est administrée en association avec un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.

**8.** Utilisation de composés de formule I selon les revendications 1-2, et/ou de sels, tautomères et stéréoisomères physiologiquement acceptables de ceux-ci, pour la préparation d'un médicament destiné au traitement de tumeurs, dans laquelle une quantité thérapeutiquement efficace d'un composé de formule I est administrée en association avec une radiothérapie et un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2008079988 A2
- WO 2008112217 A1
- WO 2008005457 A
- WO 2008124849 A
- WO 2006106326 A1
- WO 2008156726 A1
- WO 2009054941 A1
- WO 0050032 A
- US 6069134 A


### In der Beschreibung aufgeführte Nicht-Patentliteratur


- **J.S. BOEHM et al.** *Cell,* 2007, vol. 129, 1065-1079
- **S.F.EDDY et al.** *Cancer Res.,* 2005, vol. 65 (24), 11375-11383
- **C.KORHERR et al.** *PNAS,* 2006, vol. 103, 4240-4245
- **Y.CHIEN et al.** *Cell,* 2006, vol. 127, 157-170
- **D.A.BARBIE et al.** *Nature Letters,* 2009, 1-5
- *Int. J. Pharm.,* 1995, vol. 115, 61-67
- **RAFAEL CHINCHILLA ; CARMEN NÁJERA.** *Chem. Rev.,* 2007, vol. 107, 874-922
- **MORTEN MELDAL ; CHRISTIAN WENZEL TORNØE.** *Chem. Rev.,* 2008, vol. 108 (8), 2952-3015
- *Pharmaceutical Research,* 1986, vol. 3 (6), 318
- **H. KOTSUKI et al.** *J. Org. Chem.,* 1996, vol. 61, 984